(19) Europäisches Patentamt
European Patent Office
Office européen des brevets

(11) **EP 4 134 671 A1**

(12) **EUROPEAN PATENT APPLICATION**

(43) Date of publication:
**15.02.2023  Bulletin 2023/07**

(21) Application number: **21191371.0**

(22) Date of filing: **13.08.2021**

(51) International Patent Classification (IPC):
**G01N 33/574** (1985.01)    **A61K 31/00** (1974.07)

(52) Cooperative Patent Classification (CPC):
(C-Sets available)
**G01N 33/57484; A61K 31/439; A61K 45/06;
G01N 33/57426;** G01N 2800/52       (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Deutsches Krebsforschungszentrum
  Stiftung des öffentlichen Rechts
  69120 Heidelberg (DE)**
• **Universität Heidelberg
  69117 Heidelberg (DE)**

(72) Inventors:
• **Müller, Michael
  69120 Heidelberg (DE)**

• **Oehme, Ina
  69120 Heidelberg (DE)**
• **Witt, Olaf
  69120 Heidelberg (DE)**
• **Peterziel, Heike
  69120 Heidelberg (DE)**
• **Milde, Till
  69115 Heidelberg (DE)**

(74) Representative: **Altmann Stößel Dick
  Patentanwälte PartG mbB
  Theodor-Heuss-Anlage 2
  68165 Mannheim (DE)**

Remarks:
The complete document including Reference
Table(s) and the Sequence Listing(s) can be
downloaded from the EPO website

(54) **METHYLENE QUINUCLIDINONE COMPANION DIAGNOSTICS**

(57)    The present invention relates to a method for identifying a subject susceptible to treatment of cancer by administration of a methylene quinuclidinone agent, said method comprising (a) determining in a sample of said subject at least four biomarkers independently selected from the group consisting of SLC7A11, PLS3, RHBDF1, CLDN1, FAM114A1, YAP1, TJP1, SEPTIN10; COROIA, RHOH, CXCR4, PUM2, TRAF3IP3, KDM2B, PDE7A, ARHGAP9, IKZF1, ACAP1, and PDE7A; (b) comparing the result determined in step (a) to at least one reference, and (c), based on the result of step (b), identifying a subject susceptible to treatment of cancer by administration of a methylene quinuclidinone agent, and to compounds for use, kits, and devices related thereto.

**EP 4 134 671 A1**

**(Cont. next page)**

(52) Cooperative Patent Classification (CPC): (Cont.)

C-Sets
**A61K 31/439, A61K 2300/00**

**Description**

The present invention relates to a method for identifying a subject susceptible to treatment of cancer by administration of a methylene quinuclidinone agent, said method comprising (a) determining in a sample of said subject at least four biomarkers independently selected from the group consisting of SLC7A11, PLS3, RHBDF1, CLDN1, FAM114A1, YAP1, TJP1, SEPTIN10; COROIA, RHOH, CXCR4, PUM2, TRAF3IP3, KDM2B, PDE7A, ARHGAP9, IKZF1, ACAP1, and PDE7A; (b) comparing the result determined in step (a) to at least one reference, and (c), based on the result of step (b), identifying a subject susceptible to treatment of cancer by administration of a methylene quinuclidinone agent, and to compounds for use, kits, and devices related thereto.

The tumor suppressor gene TP53 is mutated in approximately 50% of all human cancers and to some extent nearly all cancers develop the ability to circumvent p53 pathway activation. Primary neuroblastomas only rarely display TP53 mutations, whereas relapsed high-risk neuroblastomas frequently show TP53 mutations and loss of p53 function, which are both linked to a poor prognosis. Over the last 20 years, novel drugs were developed to reactivate mutated p53. The most advanced drug is APR-246, which is currently being tested in 12 clinical trials. APR-246 is showing a very promising clinical potential, with a maximum plasma concentration of approximately 280 $\mu$M and a half-life of four to five hours, which corresponds to an estimated trough plasma concentration of 15-20 $\mu$M. APR-246 is the methylated functional analogue of PRIMA-1, both of which are converted spontaneously to reactive methylene quinuclidinone (MQ). MQ covalently binds to cysteine residues of the mutant p53 (namely cysteine 124 and 277), which is changing the protein's conformation. This restores its DNA-binding function and induces a strong apoptotic signal.

In addition, MQ is able to bind other cysteine residues, e.g., thioredoxin reductase 1 (TrxR1) (Peng et al. Cell death & disease 2013, 4, e881-e881) and glutathione (Liu et al., Nat Commun 2017, 8, 14844). MQ bound TrxR1 increases the ROS level via converting the enzyme to a pro-oxidant NADPH oxidase (Peng et al. 2013, loc. cit.). MQ binding impairs the antioxidant glutathione system and thus elevates the levels of reactive oxygen species (ROS) and lipid peroxidation, thereby promoting cell death (Liu et al. 2017, loc. cit.). Cysteine importers, such as SLC7A11 (also commonly known as xCT), are responsible for the intracellular amount of cysteine, and thus glutathione (Liu et al. 2017, loc. cit.). SLC7A11 gene expression was identified as a response prediction biomarker for APR-246 treatment in esophagus carcinoma cell lines, whereby low expression levels of the cysteine importer are associated with a higher response to APR-246 treatment (Liu et al. 2017, loc. cit.).

The enzyme family of HDACs is linked to oncogenic events and plays a major role in pediatric cancers of the nervous system (Koeneke et al., Cells 2015, 4, 135-168; Ridinger et al., Scientific reports 2018, 8, 10039). Hence, HDAC inhibitors demonstrated antitumor effects in various pediatric tumor models (cf. e.g. (Oehme et al., Proc Natl Acad Sci U S A 2013, 110, E2592-2601). To date, several HDAC inhibitors are approved for the treatment of hematological cancers (e.g., vorinostat for cutaneous T-cell lymphoma and panobinostat for multiple myeloma) and several clinical trials are investigating HDAC inhibitors as both single agents and in combination with chemotherapeutics, for additional tumor entities.

In view of the above, there is still a need in the art for improved means and methods for predicting susceptibility of cancer for treatment. This problem is addressed by the subject matter with the features of the independent claims. Advantageous embodiments which might be realized in an isolated fashion or in any arbitrary combinations are listed in the dependent claims.

In accordance, the present invention relates to a method for identifying a subject susceptible to treatment of cancer by administration of a methylene quinuclidinone agent comprising

(a) determining in a sample of said subject at least four biomarkers independently selected from the group consisting of SLC7A11, PLS3, RHBDF1, CLDN1, FAM114A1, YAP1, TJP1, SEPTIN10; COROIA, RHOH, CXCR4, PUM2, TRAF3IP3, KDM2B, PDE7A, ARHGAP9, IKZF1, ACAP1, and PDE7A;
(b) comparing the result determined in step a) to at least one reference, and (c) based on the result of step (b), identifying a subject susceptible to treatment of cancer by administration of a methylene quinuclidinone agent.

In general, terms used herein are to be given their ordinary and customary meaning to a person of ordinary skill in the art and, unless indicated otherwise, are not to be limited to a special or customized meaning. As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. Also, as is understood by the skilled person, the expressions "comprising a" and "comprising an" preferably refer to "comprising one or more", i.e. are equivalent to "comprising at least one". In accordance, expressions relating to one item of a plurality, unless otherwise indicated, preferably relate to at least one such item, more preferably a plurality thereof; thus, e.g. identifying "a cell" relates to identifying at least one cell, preferably to identifying a multitude of cells.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

The methods specified herein below, preferably, are in vitro methods. The method steps may, in principle, be performed in any arbitrary sequence deemed suitable by the skilled person, but preferably are performed in the indicated sequence; also, one or more, preferably all, of said steps may be assisted or performed by automated equipment. Moreover, the methods may comprise steps in addition to those explicitly mentioned above.

As used herein, if not otherwise indicated, the term "about" relates to the indicated value with the commonly accepted technical precision in the relevant field, preferably relates to the indicated value $\pm$ 20%, more preferably $\pm$ 10%, most preferably $\pm$ 5%. Further, the term "essentially" indicates that deviations having influence on the indicated result or use are absent, i.e. potential deviations do not cause the indicated result to deviate by more than $\pm$ 20%, more preferably $\pm$ 10%, most preferably $\pm$ 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. Preferably, a composition consisting essentially of a set of components will comprise less than 5% by weight, more preferably less than 3% by weight, even more preferably less than 1% by weight, most preferably less than 0.1% by weight of non-specified component(s).

The degree of identity (e.g. expressed as "%identity") between two biological sequences, preferably DNA, RNA, or amino acid sequences, can be determined by algorithms well known in the art. Preferably, the degree of identity is determined by comparing two optimally aligned sequences over a comparison window, where the fragment of sequence in the comparison window may comprise additions or deletions (e.g., gaps or overhangs) as compared to the sequence it is compared to for optimal alignment. The percentage is calculated by determining, preferably over the whole length of the polynucleotide or polypeptide, the number of positions at which the identical residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. In the context of biological sequences referred to herein, the term "essentially identical" indicates a %identity value of at least 80%, preferably at least 90%, more preferably at least 98%, most preferably at least 99%. As will be understood, the term essentially identical includes 100% identity. The aforesaid applies to the term "essentially complementary" mutatis mutandis.

The term "fragment" of a biological macromolecule, preferably of a polynucleotide or polypeptide, is used herein in a wide sense relating to any sub-part, preferably subdomain, of the respective biological macromolecule comprising the indicated sequence, structure and/or function. Thus, the term includes sub-parts generated by actual fragmentation of a biological macromolecule, but also sub-parts derived from the respective biological macromolecule in an abstract manner, e.g. in silico. In the case of determining a biomarker, it may be sufficient to determine a fragment of a specific biomarker, e.g. an epitope or other peptide fragment of a polypeptide or a subsequence of an RNA, e.g. of an mRNA; preferably, the fragment of a biomarker is specific for said biomarker, i.e. is unique for the biomarker. Unless specifically indicated otherwise herein, the compounds specified, in particular the polynucleotides and polypeptides, may be comprised in larger structures, e.g. may be covalently or non-covalently linked to further sequences.

The method for identifying a subject susceptible to treatment of cancer, preferably, is an in vitro method. Moreover, it may comprise steps in addition to those explicitly mentioned above. For example, further steps may relate, e.g., to providing a sample of the subject for step a), or providing treatment to said subject based on identification step (c). The method for identifying a subject susceptible to treatment of cancer preferably is not a diagnostic method, i.e. preferably does not diagnose cancer. Thus, the method may be preceded by diagnostic methods identifying the subject as suffering from cancer, optionally including taking a biopsy of cancer tissue.

The term "methylene quinuclidinone agent", as used herein, relates to any compound providing methylene quinuclidinone when administered to a subject. Thus, the methylene quinuclidinone agent preferably is 2-Methylen-3-quinuclidinon (also referred to as 2-Methylen-3-chinuclidinon, e.g. available as hydrochloride hydrate, 3-Methylene-4-azabicyclo[2.2.2]octan-2-one, chloride, hydrate, CAS No. 207556-03-4), or a compound converted to methylene quinuclidinone in the body of a subject, in particular APR-246 (Eprenetapopt, 2-(Hydroxymethyl)-2-(methoxymethyl)-1-azabicyclo[2.2.2]octan-3-one, CAS No. 5291-32-7) or Prima-1 (2,2-bis(hydroxymethyl)-1-azabicyclo[2.2.2]octan-3-one, CAS No. 5608-24-2). More preferably, the methylene quinuclidinone agent is APR-246. Preferably, the methylene quinuclidinone agent is an inhibitor of thioredoxin reductase 1 and/or of glutathione.

The term "subject", as referred to herein, relates to a vertebrate animal, preferably a mammal, more preferably a human. Preferably, the subject shows symptoms of cancer; symptoms of cancer and diagnostic methods related thereto are known from standard medical textbooks. More preferably, the subject has been diagnosed to suffer from cancer.

The term "sample" refers to a sample comprising separated cells or to a sample from a tissue or an organ or to a sample of a bodily fluid, preferably a sample of blood, plasma, serum, saliva, sputum, urine, of a subject, wherein the sample preferably is known or suspected to comprise tumor cells. Tissue or organ samples may be obtained from any tissue or organ by, e.g., biopsy. Preferred tissue is cancer tissue, in particular tumor tissue; thus, the sample may in particular be a biopsy of a cancer tissue, e.g. a tumor tissue. Other sources comprising cancer cells may be bodily fluids or lavage fluid, preferably in the case of non-solid cancers, or may be circulating tumor cells (CTC), which can be enriched e.g. from blood samples. Separated cells may be obtained from body fluids, such as lymph, blood, plasma, serum, liquor and others, or from the tissues or organs by separating techniques such as centrifugation or cell sorting. The sample can be obtained from the subject by routine techniques which are well known to the person skilled in the art, e.g., venous or arterial puncture or biopsy including aspiration of tissue or cellular material from a subject. For those areas which cannot be easily reached via an open biopsy, a surgery and, preferably, minimal invasive surgery can be performed.

The term "cancer", as used herein, relates to a disease of a subject, characterized by uncontrolled growth by a group of body cells ("cancer cells"). This uncontrolled growth may lead to tumor formation and may be accompanied by intrusion into and destruction of surrounding tissue (invasion) and possibly spread of cancer cells to other locations in the body (metastasis). Thus, preferably, the cancer is a solid cancer, a metastasis, and/or a relapse thereof. The cancer may, however, also be a non-solid cancer. Preferably, the cancer is a neurological cancer, preferably a brain cancer or a cancer of the peripheral nervous system, more preferably a neuroblastoma, most preferably a neuroblastoma relapse. Also preferably, the cancer is a cancer of the blood system, more preferably a lymphoma, a myeloid dysplastic syndrome, or a leukemia, still more preferably an acute lymphocytic leukemia (ALL) or an acute myeloid leukemia (AML). Preferably, the the cancer is a p53-mutated cancer and/or a cancer with impaired p53 function.

Symptoms and diagnostic methods for diagnosing the aforesaid cancers are known from standard medical textbooks.

The terms "treating" and "treatment" refer to an amelioration of the diseases or disorders referred to herein or the symptoms accompanied therewith to a significant extent. Said treating as used herein also includes an entire restoration of health with respect to the diseases or disorders referred to herein. It is to be understood that treating, as the term is used herein, may not be effective in all subjects to be treated. However, the term shall require that, preferably, a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc. Preferred confidence intervals are at least 90%, at least 95%, at least 97%, at least 98% or at least 99 %. The p-values are, preferably, 0.1, 0.05, 0.01, 0.005, or 0.0001. Preferably, the treatment shall be effective for at least 10%, at least 20% at least 50%, at least 60%, at least 70%, at least 80%, or at least 90% of the subjects of a given cohort or population. Preferably, treating cancer is reducing tumor burden and/or cancer cell load in a subject. As will be understood by the skilled person, methods and effectiveness of treatment of e.g. cancer is dependent on a variety of factors including, e.g. cancer stage and cancer type. In view of the description provided herein, treating may additionally comprise, e.g. may be preceded, accompanied, and/or followed, by surgery, chemotherapy, radiotherapy, targeted therapy, and/or immunotherapy. Also in view of the description provided herein, treating preferably comprises administration of a methylene quinuclidinone agent and may optionally comprise administration of a histone deacetylase (HDAC) inhibitor in addition to the methylene quinuclidinone agent.

The terms "radiation therapy" and "radiotherapy" are known to the skilled artisan. The terms relate to the use of ionizing radiation to treat or control cancer. The skilled person also knows the term "surgery", relating to invasive measures for treating cancer, in particular excision of tumor tissue.

As used herein, the term "chemotherapy" relates to treatment of a subject with an antineoplastic drug. Preferably, chemotherapy is a treatment including alkylating agents (e.g. cyclophosphamide), platinum (e.g. carboplatin), anthracyclines (e.g. doxorubicin, epirubicin, idarubicin, or daunorubicin) and topoisomerase II inhibitors (e.g. etoposide, irinotecan, topotecan, camptothecin, or VP16), anaplastic lymphoma kinase (ALK)-inhibitors (e.g. Crizotinib or AP26130), aurora kinase inhibitors (e.g. N-[4-[4-(4-Methylpiperazin-1-yl)-6-[(5-methyl-1H-pyrazol-3-yl)amino]pyrimidin-2-yl]sulfanylphenyl]cyclopropanecarboxamide (VX-680)), antiangiogenic agents (e.g. Bevacizumab), or Iodine131-1-(3-iodobenzyl)guanidine (therapeutic metaiodobenzylguanidine), alone or any suitable combination thereof. It is to be understood that chemotherapy, preferably, relates to a complete cycle of treatment, i.e. a series of several (e.g. four, six, or eight) doses of antineoplastic drug or drugs applied to a subject separated by several days or weeks without such application.

The term "targeted therapy", as used herein, relates to application to a patient of a chemical substance known to block growth of cancer cells by interfering with specific molecules known to be necessary for tumorigenesis or cancer or cancer cell growth. Examples known to the skilled artisan are small molecules like, e.g. PARP-inhibitors (e.g. Iniparib), or monoclonal antibodies like, e.g., Trastuzumab.

The term "immunotherapy" as used herein relates to the treatment of cancer by modulation of the immune response of a subject, e.g. as cell based immunotherapy. Said modulation may be inducing, enhancing, or suppressing said immune response. The term "cell based immunotherapy" relates to a cancer therapy comprising application of immune cells, e.g. T-cells, preferably tumor-specific NK cells, to a subject.

As used herein, the term "susceptible to treatment" relates to the property of a cancer, i.e. of cells of said cancer, to be inhibited, killed, and/or prevented from migration and/or invasion into healthy tissue by said treatment. Thus, in a subject susceptible to treatment as described herein, upon administration of an effective dose of a methylene quinuclidinone agent, said cancer preferably stops growing, more preferably is reduced in mass (partial remission), most preferably is completely removed from the subject (complete remission). Thus, preferably, in a subject susceptible to treatment cancer cells are inhibited from growing, more preferably at least partially lyse upon said treatment.

An effective dose can be established by the skilled person, the term referring to an amount of methylene quinuclidinone agent which ameliorates or treats a cancer. Therapeutic efficacy and toxicity of compounds can be determined by standard pharmaceutical procedures in cell culture or in experimental animals, e.g., by determining the ED50 (the dose therapeutically effective in 50% of the population) and/or the LD50 (the dose lethal to 50% of the population). The dose ratio between therapeutic and toxic effects is the therapeutic index, and it can be expressed as the ratio, LD50/ED50. The dosage regimen will be determined by the attending physician, preferably taking into account relevant clinical factors and, preferably, in accordance with any one of the methods described elsewhere herein. As is well known in the medical arts, a dosage for any one patient may depend upon many factors, including the patient's size, body surface area, age, the particular compound to be administered, sex, time and route of administration, general health, and other drugs being administered concurrently. Progress can be monitored by periodic assessment. A typical dose can be, for example, in the range of 1 $\mu$g to 10000 $\mu$g; however, doses below or above this exemplary range are envisioned, especially considering the aforementioned factors. However, depending on the subject and the mode of administration, the quantity of substance administration may vary over a wide range to provide from about 0.01 mg per kg body mass to about 1 mg per kg body mass, preferably. The methylene quinuclidinone agent is administered at least once in order to treat cancer. However, the methylene quinuclidinone agent may be administered more than one time, for example, preferably from one to four times, or regularly over a period of time determined by the medical practitioner. Preferably the effective dose is a dose providing a blood concentration of the methylene quinuclidinone agent of at least 10 $\mu$M, more preferably at least 20 $\mu$M, still more preferably at least 50 $\mu$M, most preferably at least 100 $\mu$M for at least 5 hours, more preferably at least 12 hours, still more preferably at least 18 hours, most preferably at least 24 hours after administration. As indicated herein above, said dosage may be repeated, e.g. on a daily basis.

Preferably, additionally metastasis, invasion, and/or remission of cancer are prevented in a subject susceptible to treatment with a methylene quinuclidinone agent, the term "preventing" referring to retaining health with respect to the diseases or disorders referred to herein for a certain period of time in a subject. It will be understood that the said period of time may be dependent on the amount of the drug compound which has been administered and individual factors of the subject discussed elsewhere in this specification. It is to be understood that prevention may not be effective in all subjects treated with the compound according to the present invention. However, the term requires that, preferably, a statistically significant portion of subjects of a cohort or population are effectively prevented from suffering from metastasis, invasion, and/or remission. Preferably, a cohort or population of subjects is envisaged in this context which normally, i.e. without preventive measures according to the present invention, would develop at least one of metastasis, invasion, and/or remission. Whether a portion is statistically significant can be determined without further ado by the person skilled in the art using various well known statistic evaluation tools discussed elsewhere in this specification.

The term "biomarker", as used herein, refers to a molecular species which serves as an indicator for a susceptibility as referred to in this specification. Said molecular species can be a metabolite itself which is found in a sample of a subject. Preferably, the biomarker is a gene product or a specific fragment thereof, in particular an RNA and/or polypeptide being the product of expression of said gene. Mechanisms of gene expression and their products are known to the skilled person from standard textbooks e.g. of cell biology. In case the biomarker, e.g. a polypeptide, has an activity, e.g. an enzymatic activity, the biomarker may also be a product of said activity. Preferably, however, the gene product is an RNA being the product of gene expression, in particular an mRNA, or is a polypeptide, preferably produced by cells of the subject from the aforesaid mRNA. Moreover, the biomarker may also be a molecular species which is derived from the aforesaid metabolite, in particular a fragment thereof. The actual metabolite may be chemically modified in the sample or during the determination process and, as a result of said modification, a chemically different molecular species, i.e. an analyte, will be the determined molecular species. Preferred chemical modifications may depend on the analytical method used and are described elsewhere herein. It is to be understood that in such a case, the analyte represents the actual metabolite and has the same potential as an indicator for the respective medical condition. Moreover, a biomarker according to the present invention is not necessarily corresponding to one molecular species. Rather, the biomarker may comprise derivatives, such as one or more splice variants, a polypeptide product of at least one splice variant, and/or fragments of a compound. Further, a biomarker can also represent the sum of variants of a gene product. Said variants may exhibit identical analytical characteristics in some cases and may, therefore, not be distinguishable by some analytical methods including those applied in the accompanying Examples described below. Thus, e.g. in RT-qPCR, all RNA molecules comprising a nucleic acid sequence to which two selected primers anneal may be detected, irrespective of whether the RNA is a full-length mRNA, a nuclear precursor, a splice variant, or a fragment of an mRNA. Also, e.g. in an immunoassay, a polypeptide may be detected as a gene product, but also any fragment or isoform of said polypeptide having the required epitope or epitopes may be detected.

The term "determining", as used herein, refers to quantitatively determining an analyte by measuring at least one characteristic feature of the analyte to be determined in a sample. Characteristic features in accordance with the present invention are features which characterize the physical and/or chemical properties including biochemical properties of an analyte. Such properties include, e.g., molecular weight, as e.g. determined by mass spectrometry; nucleic acid sequence, as e.g. determined by sequencing; capability to react with other compounds, in particular hybridization to one or more probe oligonucleotide or primer oligonucleotide(s), or immunological reactivity; capability to elicit a response in a biological read out system (e.g., induction of a reporter gene); and the like. Values for the aforesaid properties may serve as characteristic features and can be determined by techniques well known in the art. Moreover, the characteristic feature may be any feature which is derived from the values of the physical and/or chemical properties of a biomarker by standard operations, e.g., mathematical calculations such as multiplication, division or logarithmic calculus. Most preferably, the at least one characteristic feature allows the determination and/or chemical identification of the biomarker and its amount. Accordingly, the characteristic value, preferably, also comprises information relating to the abundance of the biomarker from which the characteristic value is derived. For example, a characteristic value of a biomarker may be a peak in a mass spectrum. Such a peak contains characteristic information of the biomarker, i.e. the m/z information, as well as an intensity value being related to the abundance of the said biomarker (i.e. its amount) in the sample.

A biomarker comprised by a sample may, in principle, be determined quantitatively or semi-quantitatively, preferably is determined quantitatively. For quantitative determination, either the absolute or precise amount of the biomarker will be determined or the relative amount of the biomarker will be determined based on the value determined for the characteristic feature(s) referred to herein above. The relative amount may be determined in a case where the precise amount of a biomarker can or shall not be determined. In said case, it can preferably be determined whether the amount in which the biomarker is present is increased or reduced with respect to a second sample comprising said biomarker in a second amount. Quantitatively analyzing a biomarker, thus, also includes what is sometimes referred to as semi-quantitative analysis of a biomarker. As is understood by the skilled person, the above applies to other measures of an amount of a compound, in particular a concentration, mutatis mutandis. Preferably, a biomarker is determined by a specific chemical or biological assay. Said assay shall comprise means which allow to specifically detect the at least one biomarker in the sample. Preferably, said means are capable of specifically recognizing the chemical structure of the biomarker or are capable of specifically identifying the biomarker based on its capability to react with other compounds or its capability to elicit a response in a biological read out system (e.g., induction of a reporter gene). Means which are capable of specifically recognizing the chemical structure of a biomarker are, preferably, antibodies or other proteins which specifically interact with chemical structures, such as receptors or enzymes, or aptamers. Specific antibodies, for instance, may be obtained using the biomarker as antigen by methods well known in the art. Antibodies as referred to herein include both polyclonal and monoclonal antibodies, as well as fragments thereof, such as Fv, Fab and F(ab)2 fragments that are capable of binding the antigen or hapten. The present invention also includes humanized hybrid antibodies wherein amino acid sequences of a non-human donor antibody exhibiting a desired antigen-specificity are combined with sequences of a human acceptor antibody. Moreover, encompassed are single chain antibodies and nonbodies. The donor sequences will usually include at least the antigen-binding amino acid residues of the donor but may comprise other structurally and/or functionally relevant amino acid residues of the donor antibody as well. Antibodies and their derivatives can be prepared by several methods well known in the art. Other suitable proteins which are capable of specifically recognizing a biomarker are, preferably, enzymes which are involved in the metabolic conversion of the said biomarker. Said enzymes may either use the biomarker as a substrate or may convert a substrate into the biomarker. Moreover, antibodies may be used as a basis to generate oligopeptides which specifically recognize the biomarker.

These oligopeptides shall, for example, comprise the enzyme's binding domains or pockets for the said biomarker. Suitable antibody and/or enzyme based assays may be RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA) or solid phase immune tests, in particular antibody arrays; e.g. at least four antibodies recognizing biomarkers as specified herein may be provided as an antibody array. As is understood by the skilled person, correspondingly at least four probe oligonucleotides hybridizing to biomarker RNAs may be provided as oligonucleotide array. Moreover, the biomarker may also be determined based on its capability to react with other compounds, i.e. by a specific chemical reaction.

The term "value of a biomarker", as used herein, relates to a numerical value based on an amount of a biomarker determined in a sample or a reference. Thus, preferably, the value of a biomarker is an amount of a biomarker, or is a ratio of concentrations of at least two biomarkers. Thus, as used herein, the term "determining a value of a biomarker" comprises determining the amount of the biomarker and, optionally, calculating a numerical value based on said amount.

Preferably, the biomarker is SLC7A11, also known to the skilled person as solute carrier family 7 member 11. The sequences of gene products of the human SCL7A11 are provided e.g. in Genbank Acc. Nos. NM_014331.4 (mRNA) and NP_055146.1 (protein); human SLC7A11 is also known e.g. from UniprotKB entry Q9UPY5, homologues can be found under HomoloGene entry 22684.

Preferably, the biomarker is PLS3, also known to the skilled person as plastin-3, which has several transcript variants and isoforms. The sequences of gene products of the human PLS3 are provided e.g. in Genbank Acc. Nos. NM_005032.7 (exemplary mRNA) and NP_005023.2 (exemplary protein); human PLS3 is also known e.g. from UniprotKB entry P13797, homologues can be found under HomoloGene entry 128200.

Preferably, the biomarker is RHBDF1, also known to the skilled person as rhomboid 5 homolog 1. The sequences of gene products of the human RHBDF1 are provided e.g. in Genbank Acc. Nos. NM_022450.5 (mRNA) and NP_071895.3 (protein); human RHBDF1 is also known e.g. from UniprotKB entry Q96CC6, homologues can be found under HomoloGene entry 32085.

Preferably, the biomarker is CLDN1, also known to the skilled person as claudin 1. The sequences of gene products of the human CLDN1 are provided e.g. in Genbank Acc. Nos. NM_021101.5 (mRNA) and NP_066924.1 (protein); human CLDN1 is also known e.g. from UniprotKB entry O95832, homologues can be found under HomoloGene entry 22684.

Preferably, the biomarker is FAM114A1, also known to the skilled person as family with sequence similarity 114 member A1, which has several transcript variants and isoforms. The sequences of gene products of the human FAM114A1 are provided e.g. in Genbank Acc. Nos.NM_138389.4 (exemplary mRNA) and NP_612398.2 (exemplary protein); human FAM114A1 is also known e.g. from UniprotKB entry Q8IWE2, homologues can be found under HomoloGene entry 12259.

Preferably, the biomarker is YAP1, also known to the skilled person as Yes1 associated transcriptional regulator, which has several transcript variants and isoforms. The sequences of gene products of the human YAP1 are provided e.g. in Genbank Acc. Nos. NM_006106.5 (exemplary mRNA) and NP_006097.2 (exemplary protein); human YAP1 is also known e.g. from UniprotKB entry P46937, homologues can be found under HomoloGene entry 4452.

Preferably, the biomarker is TJP1, also known to the skilled person as tight junction protein 1, which has several transcript variants and isoforms. The sequences of gene products of the human TJP1 are provided e.g. in Genbank Acc. Nos. NM_003257.5 (exemplary mRNA) and NP_003248.3 (exemplary protein); human TJP1 is also known e.g. from UniprotKB entry Q07157, homologues can be found under HomoloGene entry 2445.

Preferably, the biomarker is SEPTIN10, also known to the skilled person as septin 10, which has several transcript variants and isoforms. The sequences of gene products of the human SEPTIN10 are provided e.g. in Genbank Acc. Nos. NM_144710.5 (exemplary mRNA) and NP_653311.1 (exemplary protein); human SEPTIN10 is also known e.g. from UniprotKB entry Q9P0V9, homologues can be found under HomoloGene entry 27035.

Preferably, the biomarker is COROIA, also known to the skilled person as coronin 1A, which has several transcript variants and isoforms. The sequences of gene products of the human COROIA are provided e.g. in Genbank Acc. Nos. NM_001193333.3 (exemplary mRNA) and NP_001180262.1 (exeplary protein); human CORO1A is also known e.g. from UniprotKB entry P31146, homologues can be found under HomoloGene entry 6545.

Preferably, the biomarker is RHOH, also known to the skilled person as ras homolog family member H, which has several transcript variants and isoforms. The sequences of gene products of the human RHOH are provided e.g. in Genbank Acc. Nos. NM_001278359.2 (exemplary mRNA) and NP_001265288.1 (exemplary protein); human RHOH is also known e.g. from UniprotKB entry Q15669, homologues can be found under HomoloGene entry 3180.

Preferably, the biomarker is CXCR4, also known to the skilled person as C-X-C motif chemokine receptor 4, which has several transcript variants and isoforms. The sequences of gene products of the human CXCR4 are provided e.g. in Genbank Acc. Nos. NM_001008540.2 (exemplary mRNA) and NP_001008540.1 (exemplary protein); human CXCR4 is also known e.g. from UniprotKB entry P61073, homologues can be found under HomoloGene entry 20739.

Preferably, the biomarker is PUM2, also known to the skilled person as pumilio RNA binding family member 2, which has several transcript variants and isoforms . The sequences of gene products of the human PUM2 are provided e.g. in Genbank Acc. Nos. NM_015317.5 (exemplary mRNA) and NP_056132.1 (exemplary protein); human PUM2 is also known e.g. from UniprotKB entry Q8TB72, homologues can be found under HomoloGene entry 69183.

Preferably, the biomarker is TRAF3IP3, also known to the skilled person as TRAF3 interacting protein 3, which has several transcript variants and isoforms. The sequences of gene products of the human TRAF3IP3 are provided e.g. in Genbank Acc. Nos. NM_025228.4 (exemplary mRNA) and NP_079504.2 (exemplary protein); human TRAF3IP3 is also known e.g. from UniprotKB entry Q9Y228, homologues can be found under HomoloGene entry 11885.

Preferably, the biomarker is KDM2B, also known to the skilled person as lysine demethylase 2B, which has several transcript variants and isoforms. The sequences of gene products of the human KDM2B are provided e.g. in Genbank Acc. Nos. NM_032590.5 (exemplary mRNA) and NP_115979.3 (exemplary protein); human KDM2B is also known e.g. from UniprotKB entry Q8NHM5, homologues can be found under HomoloGene entry 13069.

Preferably, the biomarker is PDE7A, also known to the skilled person as phosphodiesterase 7A, which has several transcript variants and isoforms. The sequences of gene products of the human PDE7A are provided e.g. in Genbank Acc. Nos. NM_002603.4 (exemplary mRNA) and NP_002594.1 (exemplary protein); human PDE7A is also known e.g. from UniprotKB entry Q13946, homologues can be found under HomoloGene entry 1956.

Preferably, the biomarker is ARHGAP9, also known to the skilled person as Rho GTPase activating protein 9, which has several transcript variants and isoforms. The sequences of gene products of the human ARHGAP9 are provided e.g. in Genbank Acc. Nos. NM_032496.4 (exemplary mRNA) and NP_115885.2 (exemplary protein); human ARHGAP9 is also known e.g. from UniprotKB entry Q9BRR9, homologues can be found under HomoloGene entry 13041.

Preferably, the biomarker is IKZF1, also known to the skilled person as IKAROS family zinc finger 1, which has several transcript variants and isoforms. The sequences of gene products of the human IKZF1 are provided e.g. in Genbank Acc. Nos. NM_006060.6 (exemplary mRNA) and NP_006051.1 (exemplary protein); human IKZF1 is also known e.g. from UniprotKB entry Q13422, homologues can be found under HomoloGene entry 55948.

Preferably, the biomarker is ACAP1, also known to the skilled person as ArfGAP with coiled-coil, ankyrin repeat and PH domains 1. The sequences of gene products of the human ACAP1 are provided e.g. in Genbank Acc. Nos. NM_014716.4 (mRNA) and NP_055531.1 (protein); human ACAP1 is also known e.g. from UniprotKB entry Q15027, homologues can be found under HomoloGene entry 22835.

Preferably, the biomarker is PDE7A, also known to the skilled person as high affinity cAMP-specific 3',5'-cyclic phosphodiesterase 7A, which has several transcript variants and isoforms. The sequences of gene products of the human PDE7A are provided e.g. in Genbank Acc. Nos. NM_002603.4 (exemplary mRNA) and NP_002594.1 (exemplary protein); human PDE7A is also known e.g. from UniprotKB entry Q13946, homologues can be found under HomoloGene entry 1956.

"Comparing", as used herein, encompasses comparing the amount of a biomarker which is comprised by a sample with an amount of said biomarker in a suitable reference as specified elsewhere herein. More preferably, comparing comprises calculating a score, preferably from a multitude of biomarkers, more preferably as specified elsewhere herein. It is to be understood that comparing as referred to herein refers to a comparison of corresponding parameters or values, e.g., an absolute amount of a biomarker is compared to an absolute reference amount; a concentration of a biomarker is compared to a reference concentration; or an intensity signal obtained from the biomarker is compared to the same type of intensity signal in a reference; more preferably, a score is compared to a reference score. The comparison referred to in the methods of the present invention may be carried out manually or computer assisted. For a computer assisted comparison, the value determined may be compared to a value corresponding to a suitable reference which is stored in a database by a computer program. The computer program may further evaluate the result of the comparison by means of an expert system. Accordingly, the result of the identification referred to herein may be automatically provided in a suitable output format.

The terms "reference", "reference value", "reference amount", or "reference score", as used herein, refer to an amount of biomarker or biomarkers or a value derived therefrom which allows assessing if being susceptible to treatment of cancer by administration of a methylene quinuclidinone agent or not being susceptible is to be assumed for the subject from which the sample is derived. A suitable reference may be determined from a reference subject, preferably a population of reference subjects, preceding, simultaneously, or subsequently, with the sample. Preferably, the reference is a pre-determined reference which may be stored e.g. in a database. Also preferably, the reference is a fixed value or range of values for all subjects to be tested.

References can, in principle, be calculated for a group or cohort of subjects as specified herein based on the average or median values for a given biomarker by applying standard methods of statistics. In particular, accuracy of a test such as a method aiming to predict an event, or not, is best described by its receiver-operating characteristics (ROC) (see especially Zweig (1993), Clin. Chem. 39:561). The ROC graph is a plot of all of the sensitivity versus specificity pairs resulting from continuously varying the decision threshold over the entire range of data observed. Preferably, the reference amounts lie within the range of values that represent a sensitivity of at least 75% and a specificity of at least 45%, or a sensitivity of at least 80% and a specificity of at least 40%, or a sensitivity of at least 85% and a specificity of at least 33%, or a sensitivity of at least 90% and a specificity of at least 25%.

Preferably, the reference amount as used herein is derived from samples of a population of subjects for which it is known if they were susceptible to treatment of cancer by administration of a methylene quinuclidinone agent, or not. This reference may be a discrete figure or may be a range of figures. Evidently, the reference may vary between individual species of biomarker. The measuring system therefore may be calibrated with a sample or with a series of samples comprising known amounts of each specific biomarker. It is understood by the skilled person that in such case the amount of biomarker can preferably be expressed as arbitrary units (AU). Thus, preferably, the amounts of biomarkers are determined by comparing the signal obtained from the sample to signals comprised in a calibration curve. The amounts of biomarkers may also be obtained as arbitrary units and normalized to the amount(s) of at least one, preferably at least two, more preferably at least three, normalizer biomarkers. More preferably, a score is calculated from at least four biomarkers, preferably as specified herein below. A suitable reference may be determined as specified herein in the Examples. Also, a threshold reference, e.g. a threshold score, can be preferably used as a reference. A reference may, preferably, be derived from a subject or group of subjects being known to be or have been susceptible to treatment of cancer by administration of a methylene quinuclidinone agent. A reference amount may, preferably, also be derived from a subject or group of subjects known not be or not have been susceptible to treatment of cancer by administration of a methylene quinuclidinone agent. It is to be understood that the aforementioned amounts may vary due to statistics and errors of measurement. A deviation, i.e. a decrease or an increase of the biomarker amounts referred to herein is, preferably, a statistically significant deviation, i.e. a statistically significant decrease or a statistically significant increase.

Step (a) of the method for identifying a subject susceptible to treatment of cancer by administration of a methylene quinuclidinone agent comprises determining in a sample of said subject at least four biomarkers independently selected from the group consisting of SLC7A11, PLS3, RHBDF1, CLDN1, FAM114A1, YAP1, TJP1, SEPTIN10; COROIA, RHOH, CXCR4, PUM2, TRAF3IP3, KDM2B, PDE7A, ARHGAP9, IKZF1, ACAP1, and PDE7A.

A biomarker may be determined by any method deemed appropriate by the skilled person. The method is selected by the skilled person based on the chemical nature of the selected biomarker(s), the abundance of the biomarker(s), the desired sensitivity, and other parameters known to the skilled person. Preferably, a method is selected which allows determination of several, preferably all, biomarkers in one assay; thus, array methods (antibody arrays, nucleotide arrays) may be preferred. Preferably, the determining method is specific for the biomarker(s) of interest; thus, an assay or sub-assay (e.g. a reaction at one spot of an antibody array) preferably is specific for a given biomarker. The biomarker(s) may however also be determined by a method which as such is non-specific, but nonetheless allows quantification of the biomarker(s) of interest; such proceeding may be preferable in cases where it is desirable to provide additional information in addition to the determination of biomarkers, such as p53 and/or other cancer gene mutation status, aneuploidy, expression of further biomarker, such as surface receptors and/or hormone receptors, cancer staging markers, and the like. Thus, a biomarker may be determined e.g. by determining cancer cell transcriptome and/or proteome or parts thereof, e.g. by next-generation sequencing or by immunological methods.

Preferably, determining of a biomarker comprises performing quantitative PCR (qPCR), preferably real-time PCR as specified herein in the Examples. More preferably, said qPCR is reverse-transcription qPCR (RT-qPCR), comprising reverse transcription of RNAs into cDNA, followed by qPCR amplification of the gene product of interest. Appropriate methods for RT-qPCR and kits for performing them are known in the art and are commercially available, as are primer pairs for a biomarker of interest. Also preferably, determining a biomarker comprises performing an immunoassay, preferably as specified herein above. Preferably, the immunoassay is an ELISA, preferably comprising contacting the sample to antibodies specifically recognizing the biomarkers of interest. Appropriate methods are described in standard textbooks. Preferably, determining a biomarker comprises mass spectrometry (MS). For mass spectrometry, the analytes in the sample are ionized in order to generate charged molecules or molecule fragments. Afterwards, the mass-to-charge of the ionized analyte, in particular of the ionized biomarkers, or fragments thereof is measured. Thus, the mass spectrometry step preferably comprises an ionization step in which the biomarkers to be determined are ionized. How to apply these techniques is well known to the person skilled in the art. Moreover, suitable devices are commercially available.

Preferably, determining a biomarker comprises a sample pre-treatment step. Preferably, such pre-treatment includes treatments required to release or separate the compounds comprised in a cell or to remove excessive material or waste. Suitable techniques comprise centrifugation, extraction, fractioning, ultrafiltration, protein precipitation followed by filtration and purification, and/or enrichment of compounds. Preferably, other pre-treatments are carried out in order to provide the compounds in a form or concentration suitable for compound analysis. For example, if RT-qPCR is used in the method, it may be required to release RNA comprised in cells, to remove DNA, and/or RNases, and the like. Suitable and necessary pre-treatments depend on the means used for carrying out the method and are well known to the person skilled in the art. Samples pre-treated as described herein are also comprised by the term "sample" as specified herein.

As specified herein, at least four biomarkers independently selected from the group consisting of SLC7A11, PLS3, RHBDF1, CLDN1, FAM114A1, YAP1, TJP1, SEPTIN10; COROIA, RHOH, CXCR4, PUM2, TRAF3IP3, KDM2B, PDE7A, ARHGAP9, IKZF1, ACAP1, and PDE7A are determined. Preferably, at least two biomarkers from the group consisting of SLC7A11, PLS3, RHBDF1, CLDN1, FAM114A1, YAP1, TJP1, and SEPTIN10, (group 1 biomarkers); and at least two biomarkers from the group consisting of COROIA, RHOH, CXCR4, PUM2, TRAF3IP3, KDM2B, PDE7A, ARHGAP9, IKZF1, ACAP1, PDE7A; (group 2 biomarkers) are selected. Preferably, at least eight, more preferably at least nine, still more preferably at least ten, even more preferably at least 11, even more preferably at least 12, most preferably 13, of said biomarkers are determined. Preferably, at least the biomarkers CORO1A, RHOH, CXCR4, PUM2, TRAF3IP3, KDM2B, PDE7A, ARHGAP9, PLS3, RHBDF1, CLDN1, SLC7A11, and FAM114A1 are determined. Also preferably, at least the biomarkers IKZF1, KDM2B, PLS3, and TJP1 are determined. Also preferably, at least the biomarkers PUM2, TRAF3IP3, ACAP1, PDE7A, SEPTIN10, YAP1, TJP1, and SLC7A11 are determined. More preferably, the cancer is acute lymphocytic leukemia and at least the biomarkers IKZF1, KDM2B, PLS3, and TJP1 are determined. Also more preferably, the cancer is acute lymphocytic leukemia and at least the biomarkers PUM2, TRAF3IP3, ACAP1, PDE7A, SEPTIN10, YAP1, TJP1, and SLC7A11 are determined. Most preferably, the cancer is neuroblastoma, preferably a neuroblastoma relapse, and at least the biomarkers CORO1A, RHOH, CXCR4, PUM2, TRAF3IP3, KDM2B, PDE7A, ARHGAP9, PLS3, RHBDF1, CLDN1, SLC7A11, and FAM114A1 are determined.

The method for identifying a subject susceptible to treatment of cancer by administration of a methylene quinuclidinone agent further comprises step (b) comparing the result determined in step (a) to at least one reference.

References and methods of comparing a biomarker value or a value derived therefrom have been described herein above. Preferably, step (b) comprises calculating a sore from the biomarkers determined. Preferably, calculating said score comprises summing up the log2-transformed values determined for group 1 biomarkers and/or the log2-transformed values determined for group 2 biomarkers. More preferably, the method comprises dividing the sum of the group 2 biomarkers by the sum of the group 1 biomarkers, still more preferably comprises dividing the sum of the log2-transformed values of group 2 biomarkers by the sum of the log2-transformed values of group 1 biomarkers. Thus, preferably, the biomarkers CORO1A, RHOH, CXCR4, PUM2, TRAF3IP3, KDM2B, PDE7A, ARHGAP9, PLS3, RHBDF1, CLDN1, SLC7A11, and FAM114A1 are determined and a score is calculated according to equation (1) Score = (log2CORO1A + log2RHOH + log2CXCR4 + log2PUM2 + log2TRAF3IP3 + log2KDM2B + log2PDE7A + log2ARHGAP9) / (log2PLS3 + log2RHBDF1 + log2CLDN1 + log2SLC7A11 + log2FAM114A1) (1).

As is understood by the skilled person, in equation (1), as well as in the following equations, the designation of a biomarker stands for a value correlating with its amount in a sample determined as specified herein above.

Also preferably, the method comprises determining at least the biomarkers IKZF1, KDM2B), PLS3, and TJP1, and a score is calculated according to equation (2):

Score = (log2IKZF1+ log2KDM2B) / (log2PLS3 + log2TJP1) (2).

Also preferably, the method comprises determining at least the biomarkers PUM2, TRAF3IP3, ACAP1, PDE7A, SEPTIN10, YAP1, TJP1, and SLC7A11 and a score is calculated according to equation (3):

Score = (log2PUM2+ log2TRAF3IP3+ log2ACAP1 + log2PDE7A) / (log2SEPTIN10+ log2YAP1+ log2TJP1+ log2SLC7A11) (3).

Preferably, the cancer is AML and the method comprises determining at least the biomarkers IKZF1, KDM2B, PLS3, and TJP1, and a score is calculated according to equation (4):

Score = (log2IKZF1+ log2KDM2B) / (log2PLS3 + log2TJP1) (4).

Also preferably, the cancer is AML and the method comprises determining at least the biomarkers PUM2, TRAF3IP3, ACAP1, PDE7A, SEPTIN10, YAP1, TJP1, and SLC7A11 and a score is calculated according to equation (5):

(log2PUM2+ log2TRAF3IP3+ log2ACAP1 + log2PDE7A) / (log2SEPTIN10+ log2YAP1+ log2TJP1+ log2SLC7A11) (5).

The method for identifying a subject susceptible to treatment of cancer by administration of a methylene quinuclidinone agent further comprises step (c), based on the result of step (b), identifying a subject susceptible to treatment of cancer by administration of a methylene quinuclidinone agent.

As indicated herein above, the reference preferably is derived from a population of cells not susceptible to treatment of cancer by administration of a methylene quinuclidinone agent; in such case, preferably, a decrease of group 1 biomarkers and/or increase of group 2 biomarkers compared to said reference is indicative of a subject susceptible to treatment of cancer by administration of a methylene quinuclidinone agent, and, optionally, values of the biomarkers essentially identical to the reference are indicative of a subject not susceptible to treatment of cancer by administration of a methylene quinuclidinone agent. Also preferably, the reference is derived from a population of cells susceptible to treatment of cancer by administration of a methylene quinuclidinone agent; in such case, preferably, values of the biomarkers essentially identical to the reference are indicative of a subject susceptible to treatment of cancer by administration of a methylene quinuclidinone agent, and, optionally, an increase of group 1 biomarkers and/or a decrease of group 2 biomarkers compared to said reference is indicative of a subject not susceptible to treatment of cancer by administration of a methylene quinuclidinone agent. Preferably, a score is calculated according to equation (1) and a score higher than 1.1 is indicative of a subject susceptible to treatment of cancer by administration of a methylene quinuclidinone agent. Also, in case the subject is identified to be not susceptible to treatment of cancer by administration of a methylene quinuclidinone agent, said subject preferably is identified as a subject susceptible to combined treatment with (i) a methylene quinuclidinone and (ii) a histone deacetylase (HDAC) inhibitor.

The terms "histone deacetylase inhibitor" and "HDAC inhibitor" are known to the skilled person. Preferably, the HDAC inhibitor is an HDAC inhibitor approved for clinical use, preferably in cancer treatment. Preferably, the HDAC inhibitor is vorinostat (N-Hydroxy-N'-phenyloctanediamide, CAS No. 149647-78-9), panobinostat ((2E)-N-hydroxy-3-[4-({[2-(2-methyl-1H-indol-3-yl)ethyl]amino}methyl)phenyl]acrylamide, CAS No. 404950-80-7), entinostat ((Pyridin-3-yl)methyl ((4-[(2-aminophenyl)carbamoyl]phenyl}methyl)carbamate, CAS No. 209783-80-2), abexinostat (3-[(Dimethylamino)methyl]-N-{2-[4-(hydroxycarbamoyl)phenoxy]ethyl}-1-benzofuran-2-carboxamide, CAS No. 783355-60-2, or romidepsin ((1S,4S,7Z,10S,16E,21R)-7-Ethylidene-4,21-diisopropyl-2-oxa-12,13-dithia-5,8,20,23-tetrazabicyclo 8.7.6]tricos-16-ene-3,6,9,19,22-pentone, CAS NO. 128517-07-7).

Advantageously, it was found in the work underlying the present invention that the biomarkers as specified can be used to predict sensitivity of a cancer to treatment with a methylene quinuclidinone agent with high specificity and sensitivity.

The definitions made above apply mutatis mutandis to the following. Additional definitions and explanations made further below also apply for all embodiments described in this specification mutatis mutandis.

The present invention further relates to a methylene quinuclidinone agent for use in treatment of cancer in a subject, wherein said subject was identified to be susceptible to cancer treatment with a methylene quinuclidinone agent by the method according to the present invention.

The present invention also relates to a combined preparation comprising (i) a methylene quinuclidinone agent and (ii) an HDAC inhibitor for use in treatment of cancer in a subject, wherein said subject was identified to not be susceptible to cancer treatment with a methylene quinuclidinone agent by the method according to the present invention.

The term "combined preparation", as referred to herein, preferably comprises all pharmaceutically active compounds in one preparation so that all compounds are administered simultaneously and in the same way. Also preferably, the combined preparation comprises at least two physically separated preparations for separate administration, wherein each preparation contains at least one pharmaceutically active compound. The latter alternative is preferred in cases where the pharmaceutically active compounds of the combined preparation have to be administered by different routes, e.g. parenterally and orally, due to their chemical or physiological properties. Preferably, the at least two separated preparations are administered simultaneously. This means that the time frames of the administration of the preparations overlap. Also preferred is the sequential administration of the at least two preparations, whereas the administration of the single preparations shall occur in time frames which overlap so that the at least two pharmaceutically active compounds of the preparations are present in such plasma concentrations which enable the synergistic effect of the present invention. Preferably, the at least two preparations are administered in a time interval of at most 4 hours, 8 hours, 16 hours, 1 day, or 2 days, preferably within 1 hour, more preferably simultaneously, most preferably in a combined preparation comprising all pharmaceutically active compounds.

The present invention also relates to a kit comprising means for determining at least four biomarkers independently selected from the group consisting of COROIA, RHOH, CXCR4, PUM2, TRAF3IP3, KDM2B, PDE7A, ARHGAP9, IKZF1, ACAP1, PDE7A; PLS3, RHBDF1, CLDN1, SLC7A11, FAM114A1, TJP1, SEPTIN10, and YAP1, preferably comprised in a housing.

The term "kit", as used herein, refers to a collection of the aforementioned compounds, means or reagents which may or may not be packaged together. The components of the kit may be comprised by separate vials (i.e. as a kit of separate parts) or provided in a single vial, e.g. as a composition as specified herein above. The housing of the kit in an embodiment allows translocation of the compounds of the kit, in particular common translocation; thus, the housing may in particular be a transportable container comprising all specified components. Moreover, it is to be understood that the kit of the present invention may be used for practicing the methods referred to herein above. It is, in an embodiment, envisaged that all components are provided in a ready-to-use manner for practicing the methods referred to above. Further, the kit preferably contains instructions for carrying out said methods. The instructions can be provided by a user's manual in paper- or electronic form. For example, the manual may comprise instructions for interpreting the results obtained when carrying out the aforementioned methods using the kit. Preferably, the kit comprises further compounds, such as a reaction buffer, a hybridization solution, a lysis buffer, and the like. Preferably, the kit is adapted for use in a method of the present invention, more preferably is adapted to comprise all reagents required to perform said method or methods. Preferably, the kit comprises means for determining the biomarkers as specified herein as the only means for determining a biomarker; thus, the kit preferably is specific for the identification and/or a method as specified herein.

The term "means for determining a biomarker" is understood by the skilled person to relate to a means adapted for determining said biomarker, preferably in a sample as specified herein above. Thus, the selection of a means for determining may in particular depend on the detection method envisaged, e.g. qPCR, in which case the means may be at least one oligonucleotide per biomarker, or immunologic detection, in which case the means may be at least one antibody per biomarker. Preferred means for determining a biomarker as specific means, i.e. means specifically only detecting one biomarker.

The present invention also relates to a method of treating cancer in a subject comprising (A) identifying a subject susceptible to treatment of cancer by administration of a methylene quinuclidinone agent by the method according to the present invention; and

(B1) treating said subject identified as a subject susceptible to treatment of cancer by administration of a methylene quinuclidinone agent, preferably by administration of a methylene quinuclidinone agent as sole reactive oxygen inducing agent; and/or

(B2) treating said subject identified as a subject not susceptible to treatment of cancer by administration of a methylene quinuclidinone agent by administration of (i) a methylene quinuclidinone agent and (ii) an HDAC inhibitor, or by an alternative cancer therapy.

The aforesaid method of the present invention, preferably, is an in vivo method with regard to the treatment steps. Moreover, it may comprise steps in addition to those explicitly mentioned above. Moreover, one or more of said steps may be assisted or performed by automated equipment.

The present invention also relates to a method comprising determining at least four biomarkers independently selected from the group consisting of SLC7A11, PLS3, RHBDF1, CLDN1, FAM114A1, YAP1, TJP1, SEPTIN10; COROIA, RHOH, CXCR4, PUM2, TRAF3IP3, KDM2B, PDE7A, ARHGAP9, IKZF1, ACAP1, and PDE7A, in a biological sample.

The aforesaid method of the present invention, preferably, is an in vitro method. Moreover, it may comprise steps in addition to those explicitly mentioned above. Moreover, one or more of said steps may be assisted or performed by automated equipment.

The present invention also relates to a device comprising an evaluation unit with a processor, and, preferably tangibly embedded, instructions which, when performed on the processor, cause the device to perform at least step (b) of the method according to the present invention.

The term "device", as used herein, relates to a system of means comprising at least the aforementioned means operatively linked to each other as to allow the determination. Typical means for carrying out the determination are disclosed herein above. An evaluation unit is any means capable of providing the analysis as specified; preferably, the evaluation unit is or comprises a data processing means, such as a microprocessor, a handheld device such as a mobile phone, or a computer. How to link the means in an operating manner will depend on the type of means included into the device. Preferably, the means are comprised by a single device. Preferably, the instructions and interpretations are comprised in an executable program code comprised in the device, such that, as a result of determination, an identification of a subject at risk of developing cancer or not may be output to a user. Typical devices are those which can be applied without the particular knowledge of a specialized technician, e.g., electronic devices which merely require loading data, e.g. results of step (a) of the method. The output may be given as output of raw data which need interpretation by a technician. preferably, the output of the device is, however, processed, i.e. evaluated, raw data, the interpretation of which does not require a technician. Preferably, the the device further comprises a memory unit, preferably comprising a database comprising at least one reference value for a biomarker or a score derived therefrom. Also preferably, the device further comprises a data input device, allowing input of biomarker values determined in step (a) of the method.

The present invention moreover relates to a system comprising the device according to the present invention and a measuring unit adapted for measuring the amount of at least one biomarker specified in the method according to the present invention.

The term "system", as used herein, relates to a system of means comprising at least the aforementioned means operatively linked to each other as to allow the result of the detection to be obtained. Preferred means for determining biomarkers are disclosed herein above in connection with the methods of the invention. How to link the means in an operating manner will depend on the type of means included into the system. In an embodiment, the means of the system are comprised by a single housing. Preferably, the measuring unit comprises a receptacle for a sample. The receptacle may directly contact the sample, or may be a receptacle for a further means receiving the sample, wherein the further means may be e.g. a multi-well plate, to which a sample or a multiplicity of samples may be applied. Moreover, the sample treatment unit preferably comprises a reservoir connected to a dosing means, e.g. a tubing connected to a pump, preferably comprising a lysis buffer. Preferably, the measuring unit comprises at least one detector compound, e.g. in a dried form or in a reservoir connected to a dosing means, e.g. a tubing connected to a pump. Preferably, the measuring unit further comprises a detection unit for detecting a biomarker. Means suitable as a detection unit according to the present invention are known to the skilled person. Typical means for determining biomarkers, and means for carrying out the determination are disclosed herein above. As is understood by the skilled person, means for determining biomarkers include means capable for determining an amount of a biomarkers, such as a quantitative PCR unit or an MS unit, or means for determining biomarkers activity, such as an optical unit detecting a signal of a reporter gene assay. Further typical systems comprise the detection units (e.g., biosensors, arrays, solid supports coupled to ligands specifically recognizing the biomarker, Plasmon surface resonance devices, NMR spectrometers, mass-spectrometers etc.).

In view of the above, the following embodiments are particularly envisaged:

Embodiment 1: A method for identifying a subject susceptible to treatment of cancer by administration of a methylene quinuclidinone agent, said method comprising

(a) determining in a sample of said subject at least four biomarkers independently selected from the group consisting of SLC7A11, PLS3, RHBDF1, CLDN1, FAM114A1, YAP1, TJP1, SEPTIN10; COROIA, RHOH, CXCR4, PUM2, TRAF3IP3, KDM2B, PDE7A, ARHGAP9, IKZF1, ACAP1, and PDE7A;
(b) comparing the result determined in step (a) to at least one reference, and
(c), based on the result of step (b), identifying a subject susceptible to treatment of cancer by administration of a methylene quinuclidinone agent.

Embodiment 2: The method of embodiment 1, wherein said at least four biomarkers are selected by independently selecting at least two biomarkers from the group consisting of SLC7A11, PLS3, RHBDF1, CLDN1, FAM114A1, YAP1, TJP1, and SEPTIN10, (group 1 biomarkers); and independently selecting at least two biomarkers from the group consisting of COROIA, RHOH, CXCR4, PUM2, TRAF3IP3, KDM2B, PDE7A, ARHGAP9, IKZF1, ACAP1, and PDE7A (group 2 biomarkers).

Embodiment 3: The method of embodiment 1 or 2, wherein said method comprises determining at least eight, preferably at least nine, more preferably at least ten, even more preferably at least 11, still more preferably at least 12, most preferably 13, of said biomarkers.

Embodiment 4: The method of any one of embodiments 1 to 3, wherein said method comprises

(i) determining the biomarkers COROIA, RHOH, CXCR4, PUM2, TRAF3IP3, KDM2B, PDE7A, ARHGAP9, PLS3, RHBDF1, CLDN1, SLC7A11, and FAM114A1;
(ii) determining the biomarkers IKZF1, KDM2B, PLS3, and TJP1; or
(iii) determining the biomarkers PUM2, TRAF3IP3, ACAP1, PDE7A, SEPTIN10, YAP1, TJP1, and SLC7A11.

Embodiment 5: The method of any one of embodiments 1 to 4, wherein said methylene quinuclidinone agent is an inhibitor of thioredoxin reductase 1 and/or of glutathione.

Embodiment 6: The method of any one of embodiments 1 to 5, wherein said methylene quinuclidinone agent is APR-246 (Eprenetapopt, 2-(Hydroxymethyl)-2-(methoxymethyl)-1-azabicyclo[2.2.2]octan-3-one, CAS No. 5291-32-7) or Prima-1 (2,2-bis(hydroxymethyl)-1-azabicyclo[2.2.2]octan-3-one, CAS No. 5608-24-2).

Embodiment 7: The method of any one of embodiments 1 to 6, wherein said methylene quinuclidinone agent is APR-246.

Embodiment 8: The method of any one of embodiments 1 to 7, wherein said cancer is neuroblastoma, lymphoma, acute lymphocytic leukemia, acute myeloid leukemia, or myeloid dysplastic syndrome.

Embodiment 9: The method of any one of embodiments 1 to 8, wherein said cancer is neuroblastoma, preferably a neuroblastoma relapse.

Embodiment 10: The method of any one of embodiments 1 to 8, wherein said cancer is acute lymphocytic leukemia and wherein at least the biomarkers IKZF1, KDM2B, PLS3, and TJP1 are determined.

Embodiment 11: The method of any one of embodiments 1 to 8, wherein said cancer is acute lymphocytic leukemia and wherein at least the biomarkers PUM2, TRAF3IP3, ACAP1, PDE7A, SEPTIN10, YAP1, TJP1, and SLC7A11 are determined.

Embodiment 12: The method of any one of embodiments 1 to 11, wherein the cancer is a p53-mutated cancer.

Embodiment 13: The method of any one of embodiments 1 to 12, wherein said reference is derived from a population of cells not susceptible to treatment of cancer by administration of a methylene quinuclidinone agent.

Embodiment 14: The method of embodiment 13, wherein a decrease of group 1 biomarkers and/or increase of group 2 biomarkers compared to said reference is indicative of a subject susceptible to treatment of cancer by administration of a methylene quinuclidinone agent, and, optionally, wherein values of the biomarkers essentially identical to the reference are indicative of a subject not susceptible to treatment of cancer by administration of a methylene quinuclidinone agent.

Embodiment 15: The method of any one of embodiments 1 to 14, wherein said reference is derived from a population of cells susceptible to treatment of cancer by administration of a methylene quinuclidinone agent.

Embodiment 16: The method of embodiment 15, wherein values of the biomarkers essentially identical to the reference are indicative of a subject susceptible to treatment of cancer by administration of a methylene quinuclidinone agent, and, optionally, wherein an increase of group 1 biomarkers and/or a decrease of group 2 biomarkers compared to said reference is indicative of a subject not susceptible to treatment of cancer by administration of a methylene quinuclidinone agent.

Embodiment 17: The method of any one of embodiments 1 to 16, wherein said method comprises calculating a score based on the results of the determination in step (a). Embodiment 18: The method of embodiment 17, wherein calculating said score comprises summing up the log2-transformed values determined for group 1 biomarkers and/or the log2-transformed values determined for group 2 biomarkers.

Embodiment 19: The method of embodiment 17 or 18, wherein said method comprises dividing the sum of the group 2 biomarkers by the sum of the group 1 biomarkers.

Embodiment 20: The method of any one of embodiments 17 to 19, wherein the biomarkers COROIA, RHOH, CXCR4, PUM2, TRAF3IP3, KDM2B, PDE7A, ARHGAP9, PLS3, RHBDF1, CLDN1, SLC7A11, and FAM114A1 are determined and wherein the score is calculated according to equation (1)

$$Score= (log2CORO1A + log2RHOH + log2CXCR4 + log2PUM2 + log2TRAF3IP3 + log2KDM2B + log2PDE7A + log2ARHGAP9) / (log2PLS3 + log2RHBDF1 + log2CLDN1 + log2SLC7A11 + log2FAM114A1) \quad (1).$$

Embodiment 21: The method of embodiment 20, wherein a score of greater than 1.1 is indicative of a subject susceptible to treatment of cancer by administration of a methylene quinuclidinone agent.

Embodiment 22: The method of any one of embodiments 1 to 21, wherein the subject identified to be not susceptible to treatment of cancer by administration of a methylene quinuclidinone agent is identified as a subject susceptible to combined treatment with (i) a methylene quinuclidinone agent and (ii) a histone deacetylase (HDAC) inhibitor.

Embodiment 23: The method of embodiment 22, wherein the HDAC inhibitor is vorinostat, panobinostat, entinostat, abexinostat, or romidepsin.

Embodiment 24: The method of any one of embodiments 1 to 23, wherein in step (a) the amount and/or activity of said biomarkers is determined.

Embodiment 25: The method of any one of embodiments 1 to 24, wherein said sample is a cancer sample, preferably a tumor sample.

Embodiment 26: A methylene quinuclidinone agent for use in treatment of cancer in a subject, wherein said subject was identified to be susceptible to cancer treatment with a methylene quinuclidinone agent by the method according to any one of embodiments 1 to 25. Embodiment 27: A combined preparation comprising (i) a methylene quinuclidinone agent and (ii) an HDAC inhibitor for use in treatment of cancer in a subject, wherein said subject was identified to not be susceptible to cancer treatment with a methylene quinuclidinone agent by the method according to any one of embodiments 1 to 25.

Embodiment 28: A kit comprising means for determining at least four biomarkers independently selected from the group consisting of COROIA, RHOH, CXCR4, PUM2, TRAF3IP3, KDM2B, PDE7A, ARHGAP9, IKZF1, ACAP1, PDE7A; PLS3, RHBDF1, CLDN1, SLC7A11, FAM114A1, TJP1, SEPTIN10, and YAP1, preferably comprised in a housing.

Embodiment 29: A method of treating cancer in a subject comprising

(A) identifying a subject susceptible to treatment of cancer by administration of a methylene quinuclidinone agent by the method according to any one of embodiments 1 to 25; and

(B1) treating said subject identified as a subject susceptible to treatment of cancer by administration of a methylene quinuclidinone agent, preferably by administration of a methylene quinuclidinone agent as sole reactive oxygen inducing agent; and/or

(B2) treating said subject identified as a subject not susceptible to treatment of cancer by administration of a methylene quinuclidinone agent by administration of (i) a methylene quinuclidinone agent and (ii) an HDAC inhibitor, or by an alternative cancer therapy.

Embodiment 30: A method comprising determining at least four biomarkers independently selected from the group consisting of SLC7A11, PLS3, RHBDF1, CLDN1, FAM114A1, YAP1, TJP1, SEPTIN10; COROIA, RHOH, CXCR4, PUM2, TRAF3IP3, KDM2B, PDE7A, ARHGAP9, IKZF1, ACAP1, and PDE7A, in a biological sample.

Embodiment 31: A device comprising an evaluation unit with a processor, and, preferably tangibly embedded, instructions which, when performed on the processor, cause the device to perform at least step (b) of the method according to any one of embodiments 1 to 24.

Embodiment 32: A system comprising a device according to embodiment 31 and a measuring unit adapted for measuring the amount of at least one biomarker specified in the method according to any one of embodiments 1 to 24.

All references cited in this specification are herewith incorporated by reference with respect to their entire disclosure content and the disclosure content specifically mentioned in this specification.

Figure Legends

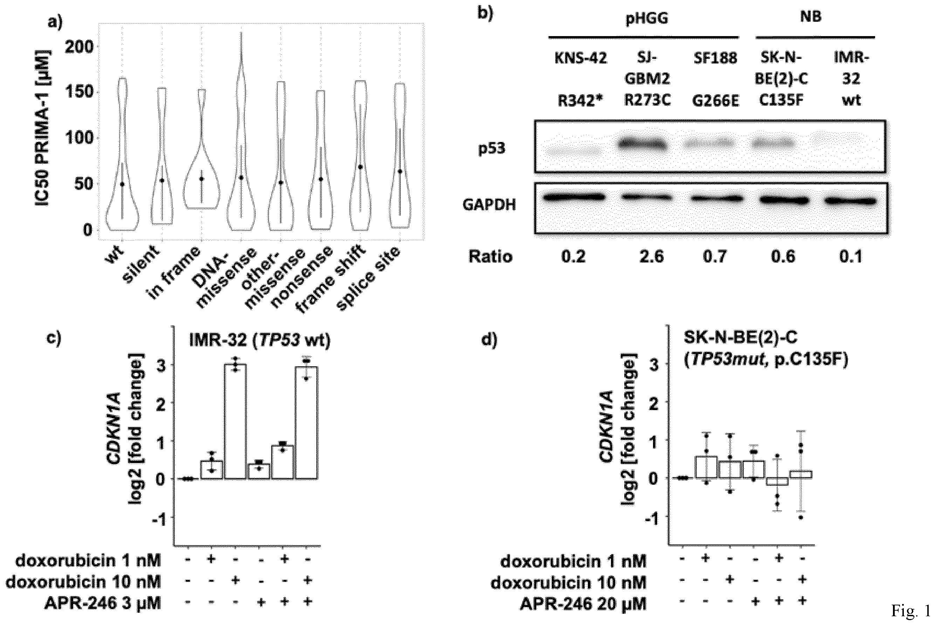

**Figure 1**. *TP53* mutation status insufficiently predicts the APR-246/PRIMA-1 response. (**a**): PRIMA-1 sensitivity separated according to *TP53* mutation status derived from Cancer Cell Line Encyclopedia and CTD$^2$ portal (n=805); (**b**): Expression of p53 is shown in the Western blot of the whole cell lysates of one *TP53* nonsense mutated cell line (KNS-42), three *TP53* missense mutated cell lines (SJ-GBM2, SF188, SK-N-BE(2)-C) and one *TP53* wild type cell line (IMR-32). GAPDH served as loading control. The ratio indicates normalization of p53 to the GAPDH signal; (**c**) and (**d**): RT-PCR measurement of *TP53* pathway activation. *CDKN1A* expression is depicted as log2 transformed fold change to solvent control. Treatment was applied for 24 h with doxorubicin or APR-246, alone or in combination, as indicated.

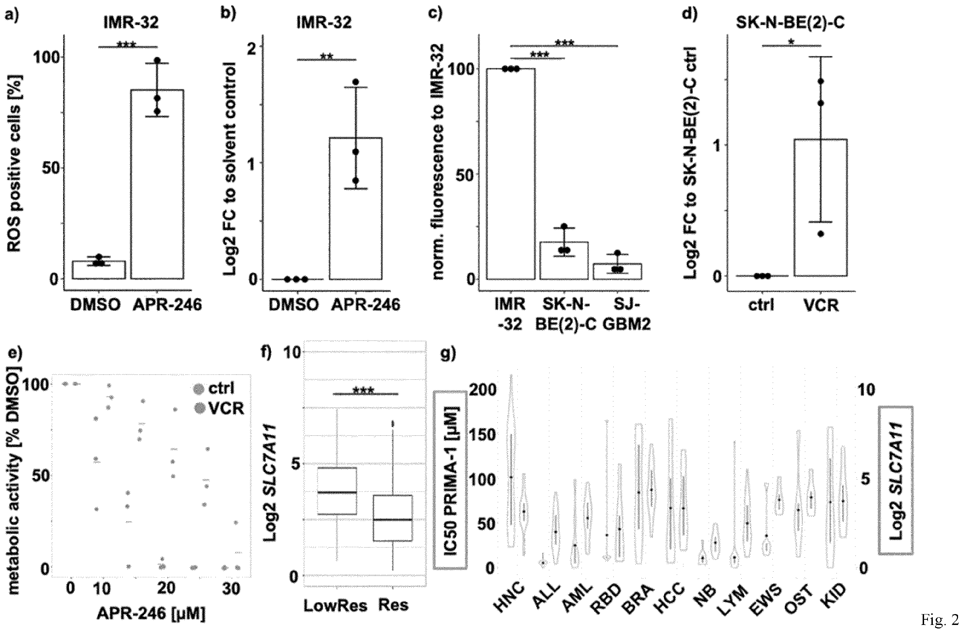

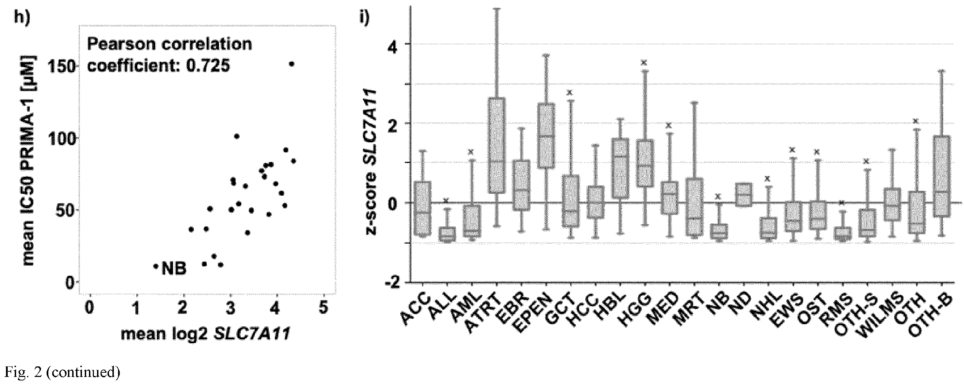

**Figure 2.** APR-246 response depends on the ROS levels. (**a**): The percentage of ROS positive IMR-32 cells after treatment with 50 $\mu$M APR-246 for 5 h compared to solvent control (DMSO) detected after DCFDA staining by FACS; (**b**): Log2 fold change of BODIPY positive IMR-32 cells after treatment with 50 $\mu$M APR-246 for 5 h compared to solvent control (DMSO) detected by FACS; (**c**): Basal ROS level in IMR-32, SK-N-BE(2)-C and SJ-GBM2 detected after DCFDA staining by FACS. Data were normalized to IMR-32 signal; (**d**): Log2 fold change of basal ROS level in vincristine resistant SK-N-BE(2)-C compared to control SK-N-BE(2)-C detected after DCFDA staining by FACS; (**e**): Metabolic activity measurement with CellTiter Glo-assay in vincristine resistant SK-N-BE(2)-C (red) and control SK-N-BE(2)-C (blue) after 72 h treatment with APR-246 0 - 30 $\mu$M. Luminescence was normalized to the respective solvent control (DMSO); (**f**): Log2 *SLC7A11* expression of PRIMA-1 less responsive cell lines (LowRes, IC50 < 20 $\mu$M) compared to responsive cell lines (Res, IC50 > 20 $\mu$M) from the Cancer Cell Line Encyclopedia (n=744); (**g**): Combination of log2 *SLC7A11* expression and PRIMA-1 sensitivity stratified by cancer cell line entities with data obtained from Cancer Cell Line Encyclopedia and CTD$^2$ portal (n=229); (**h**): Plot of the mean log2 SLC7A11 expression of entities (n=26) from the CCLE against the mean PRIMA-1 IC50 of the respective entities. Pearson correlation coefficient was calculated using R software; (**i**) Z-scores of *SLC7A11* log2 expression from INFORM patient samples stratified by entities and analyzed with R2 (n=1643). **ACC:** adrenocortical carcinoma, **ALL:** acute lymphoblastic leukemia, **AML:** acute myeloid leukemia, **ATRT:** atypical teratoid/rhabdoid tumor, **BRA:** Brain cancer, **EBR:** embryonal brain tumor, **EPEN:** ependymoma, **GCT**: germ cell tumor, **HCC:** hepatocellular carcinoma, **HBL:** hepatoblastoma, **HCC:** Liver cancer, **HGG:** high-grade glioma, **HNC:** Head and Neck Cancer, **KID:** Kidney Cancer, **LYM:** Lymphoma, **MED:** medulloblastoma, **MRT**: malignant rhabdoid tumor, **NB:** neuroblastoma, **ND**: not defined, **NHL:** non-Hodgkin lymphoma, **EWS:** Ewing sarcoma, **OST:** osteosarcoma, **RBD**: Rhabdoid, **RMS**: rhabdomyosarcoma, **OTHS:** other sarcoma, **WILMS:** nephroblastoma, **OTH:** other, **OTHB:** other brain tumor.

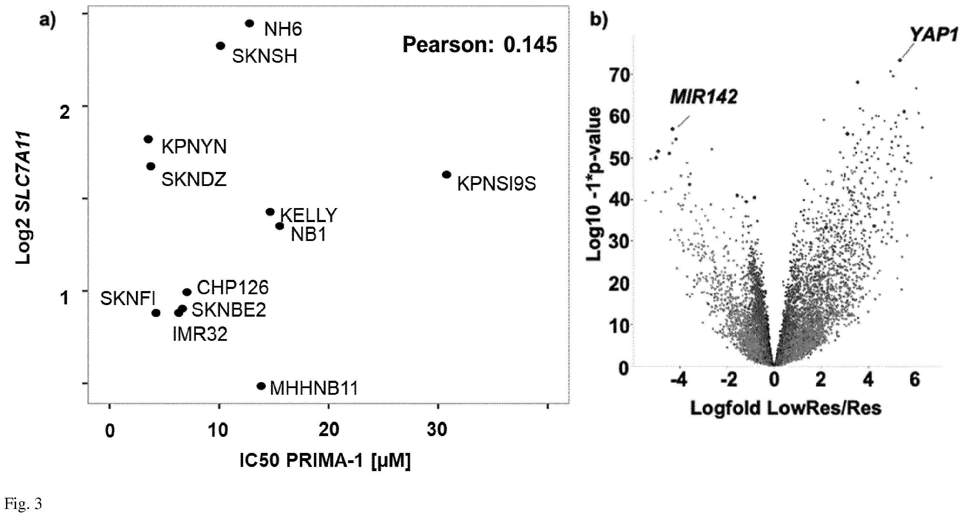

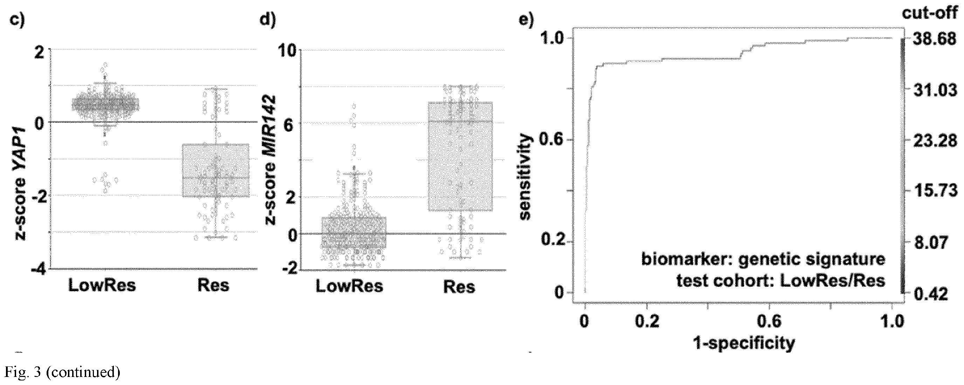

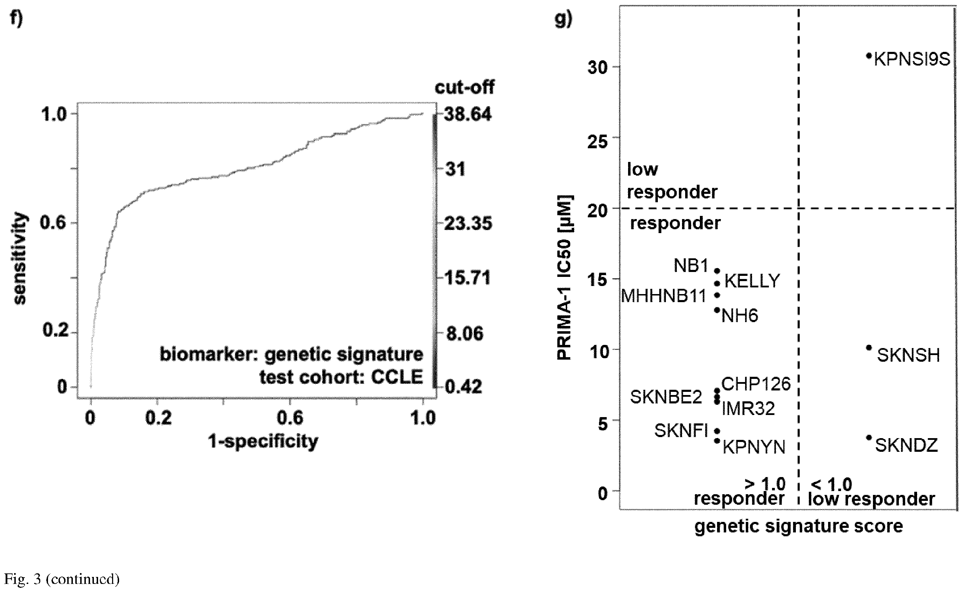

**Figure 3.** Identification of a biomarker predicting APR-246 responsiveness (**a**): in silico analysis of log2 *SLC7A11* and PRIMA-1 sensitivity (IC50 in $\mu$M) with data obtained from Cancer Cell Line Encyclopedia and CTD$^2$ portal for available neuroblastoma cell lines. The Pearson correlation coefficient (0.145) was calculated using R software (n=12); (**b**): volcano plot of differentially expressed genes between less responsive cell lines (PRIMA-1 IC50 app. > 40 $\mu$M, n=303) and responsive cell lines (PRIMA-1 IC50 app. < 10 $\mu$M, n=102) calculated using R2 software. Except for *YAP1* and *MIR142* (most significantly down and up-regulated gene respectively) all red dots indicate genes that are part of the genetic signature; (**c**) and (**d**): boxplot of z-score of in responsive cell lines that were the most significantly downregulated (**c**, *YAP* ) and upregulated (**d**, *MIR142*) genes plotted with R2; (**e**) and (**f**): receiver operating characteristic (ROC) curve calculated with ROCR package in R software for the genetic signature score to discriminate between low-responders (PRIMA-1 IC50 app. > 40 $\mu$M, n=303) and responders (PRIMA-1 IC50 app. < 10 $\mu$M, n=102) (**e**) or to discriminate the IC50s less than or greater than 20 $\mu$M (**f**). Responder and low-responder status (n=405) (**e**) or the whole CCLE cell lines (n=711) with known PRIMA-1 sensitivity data were used as test cohort (**f**); (**g**): in silico analysis of genetic signature score predicting PRIMA-1 responsiveness in neuroblastoma sub-cohort from CCLE (n=12). PRIMA-1 sensitivity cut-off 20 $\mu$M was chosen. Ten out of twelve cell lines were predicted correctly. SK-N-SH and SK-N-DZ were falsely predicted to be low-responders. **LowRes:** low-responder (n=303), **Res:** responder (n=102).

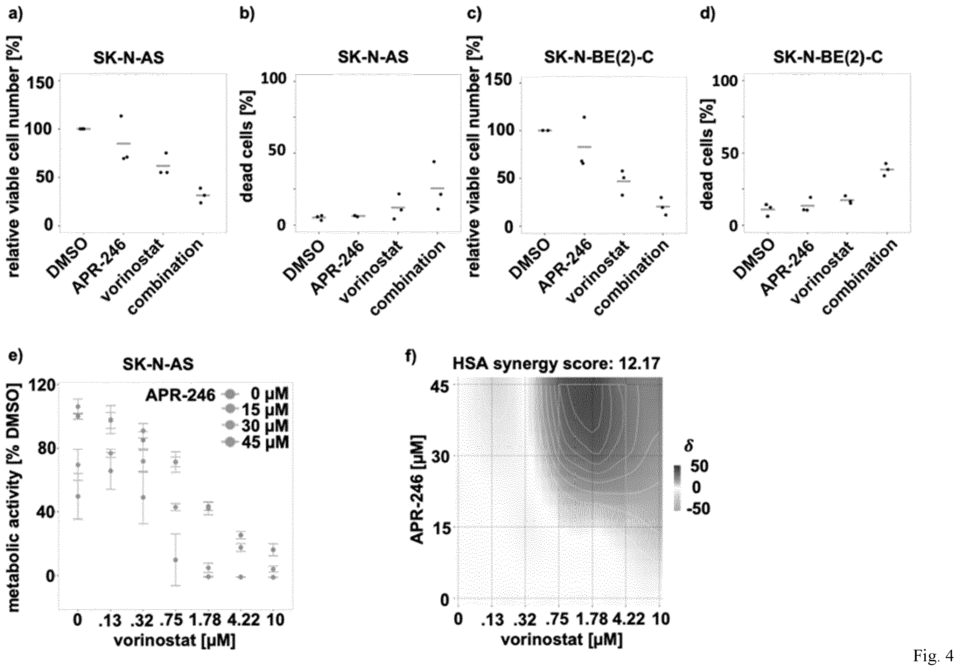

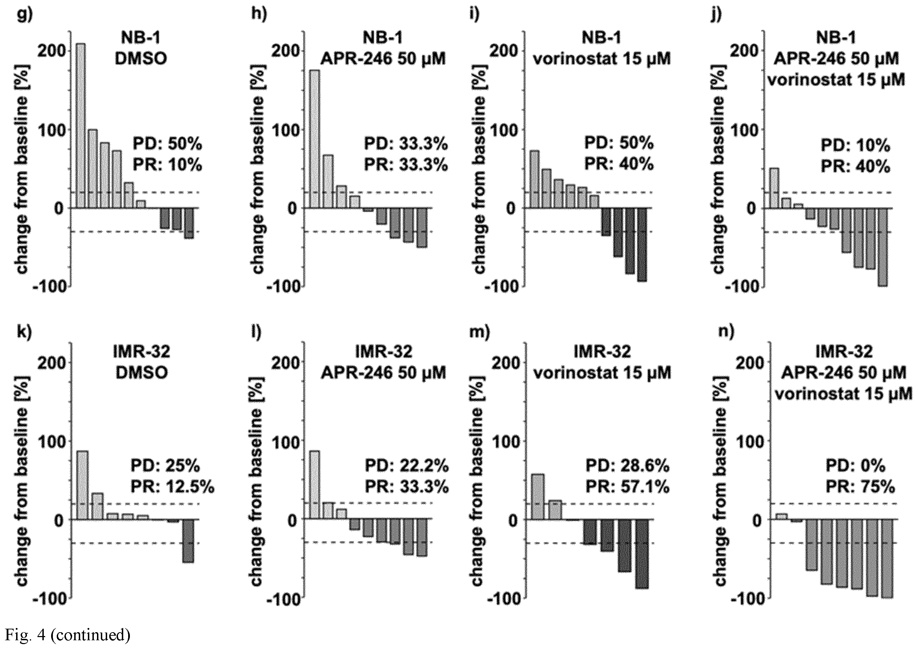

**Figure 4.** Combining APR-246 with HDAC inhibition effectively sensitizes neuroblastoma in vitro and in vivo. Determination of viable cell number/ml normalized to solvent (DMSO)-treated cells (a), (c) and dead cell count (b), (d) after automated Trypan blue exclusion assay of neuroblastoma cell lines SK-N-AS and SK-N-BE(2)-C. Cells were pretreated with 0.5 $\mu$M vorinostat for 72 h and afterwards treated with 20 $\mu$M APR-246 for 24 h. Bars indicate control, single and sequential treatment; (**e**) and (**f**): metabolic activity measurement via CellTiter Glo-assay in neuroblastoma cell line SK-N-AS after 72 h treatment with 0 $\mu$M - 10 $\mu$M vorinostat combined with fixed APR-246 concentrations

of 0 μM, 15 μM, 30 μM and 45 μM. Normalization was performed to the solvent control (DMSO). Synergy calculation (**f**) was performed with synergyfinder (synergyfinder.fimm.fi) by applying HSA (highest single agent) model; (**g**), (**h**), (**i**), (**j**), (**k**), (**l**), (**m**) and (**n**): waterfall plots demonstrating the change in tumor volume (%) for each individual xenograft, from baseline (day 1 = start of the treatment) to day 3 after yolk sac-implantation of NB-1 ((**g**), (**h**), (**i**), (**j**)) and IMR-32 (**k**), (**l**), (**m**), (**n**)) cells. Dotted lines are drawn according to Response Evaluation Criteria in Solid Tumors (RECIST) 1.1 adopted for zebrafish tumors, to visualize the best response: progressive disease (PD), at least a 20% increase in tumor volume; partial response (PR), at least a 30% decrease in tumor volume; each bar reflects one individual xenograft. The following treatments were applied: APR-246: 50 μM; vorinostat: 15 μM and combination.

The following Examples shall merely illustrate the invention. They shall not be construed, whatsoever, to limit the scope of the invention.

**Example 1: Materials and Methods**

*1.1 Cell Culture*

Cell lines were cultured at 37°C in a humidified atmosphere with 5% $CO_2$. DNA fingerprinting authentication (DSMZ, Braunschweig, Germany) and bacterial and viral (including mycoplasma) contaminations were regularly checked (Multiplexion, Heidelberg, Germany). Adherent cell lines were detached using Trypsin (Gibco, Thermo Fisher Scientific Inc., Waltham, MA, USA).

The following neuroblastoma cell lines (all passage < 30) were cultured in Dulbecco's Modified Eagle Medium (DMEM, Gibco) with L-Glutamine supplemented with 10% fetal calf serum (FCS, Sigma-Aldrich, Munich, Germany) and 1% non-essential amino acids (NEAA, Lonza, Basel, Switzerland): SK-N-BE(2)-C (European Collection of Cell Cultures, ECACC), IMR-32 (German Collection of Microorganisms and Cell Cultures, DSMZ, Darmstadt, Germany), SK-N-AS (kindly provided by M. Schwab, DKFZ ), SH-SY5Y (DSMZ). SK-N-FI (kindly provided by F. Westermann, DKFZ), Kelly (DSMZ), IMR5/75 (kindly provided by F. Westermann, DKFZ), SIMA (kindly provided by F. Westermann, DKFZ), SH-EP (passage 15 to 30, kindly provided by F. Westermann, DKFZ), SK-N-BE(2) (passage 15 to 30, DSMZ) and NB-1 (passage 15 to 30, RIKEN Cell Bank, Japan) were cultured using RPMI 1640 with L-Glutamine (Gibco), 10% FCS, 1% NEAA.

Vincristine resistant and non-resistant SK-N-BE(2)-C (passage 30 to 45, kindly provided by M. Michaelis, University of Kent, and J. Cinatl, Goethe University, Frankfurt am Main) were cultured using IMDM with L-Glutamine, 2mM HEPES (Gibco) supplemented with 10% FCS; for the resistant cells, 20 ng/ml vincristine was added to the medium. The cells were made to be resistant to vincristine-treatment through long-term cultivation in a vincristine containing medium.

The following pediatric high-grade glioma cell lines were used: SJ-GBM2 (passage 30 to 45, DMEM, 10% FCS, 1% NEAA), KNS-42 (passage 55 to 70, DMEM, 10% FCS, 1% NEAA), and SF188 (passage 95 to 110, DMEM, 10% FCS).

For the culture of Δ6RT 3T3 and C5-G1 3T3 (passage 5 to 20 after obtaining, kindly provided by D. Pestov, Department of Cell Biology and Neuroscience, Rowan University School of Osteopathic Medicine, Stratford, NJ, 08084, USA), DMEM supplemented with 10% FCS was used.

The T-cell leukemia cell line Jurkat (passage 5 to 15 after obtaining, kindly provided by J. Hoheisel, DKFZ) was cultured using RPMI 1640 with L-Glutamine (Gibco), 10% FCS, 1% NEAA. The ALL cell lines NALM-6 (passage 5 to 15 after obtaining, kindly provided by T. Grünewald, DKFZ) and REH (passage 5 to 15, kindly provided by T. Grünewald, DKFZ) and the B-cell lymphoma cell line Jeko-1 (passage 5 to 15, kindly provided by M. Persicke, lab of D. Mertens, DKFZ) were cultured using RPMI 1640 with L-Glutamine (Gibco) supplemented with 10% FCS (Sigma-Aldrich). The T-cell lymphoma cell line HuT 78 was cultured with IMDM with L-Glutamine, and 2mM HEPES (Gibco) supplemented with 20% FCS.

Long-term cultures (LTC) were established from primary tumor samples obtained through the INFORM study (INdividualized Therapy FOr Relapsed Malignancies in Childhood). The cells (passage 15 to 30 after isolation from the original tumor) were cultured using Tumor Stem Medium (TSM). The TSM consisted of filtered (Stericup-GP 500ml Express Plus PES 0.22 μm, Merck, Darmstadt, Germany) TSM Base Medium (47.5% Neurobasal-A Medium, 47.5% D-MEM/F-12, 1% HEPES Buffer Solution (1M), 1% sodium pyruvate MEM 100MM(CE), 1% MEM Non-Essential Amino Acids Solution 10mM, L-glutamine solution BIOXTRA, 2 mM, 1% Penicillin-Streptomycin-Glutamine, Life Technologies, Thermo Fisher Scientific Inc.) supplemented with 2% B27 Supplement Minus Vitamin A (50X, Life Technologies, Thermo Fisher Scientific Inc.), 0.02% (w/v) recombinant human EGF (100 μg/ml, PeproTech Inc., Rocky Hill, New Jersey, USA), 0.02% (w/v) recombinant human FGF-basic (100 μg/ml, PeproTech Inc.), 0.05% H-PDGF-AA (20 μg/ml, PeproTech Inc.) and 0.1% Heparin Solution (2mg/ml, Sigma-Aldrich). The following cultures from INFORM patient samples were used: INF_R_1288_LTC, rhabdoid tumor (ATRT, MYC by methylation profiling); INF_R_1467_LTC, soft tissue sarcoma (eRMS by methylation profiling); INF R 1490 LTC, osteosarcoma (OS_HG by methylation profiling) and INF_R_1632_PDX_LTC, neuroblastoma (NB, MYCN by methylation profiling). Ethics committee approval for INFORM was obtained from Heidelberg University Hospital's review board.

Isolation and long-term cell culture establishment of PDX_LTC cells: INF_R_1632_PDX cells were isolated from a subcutaneous patient-derived xenograft (PDX) model established with a fresh surgical specimen of an INFORM patient with relapsed neuroblastoma according to a protocol adapted from Stewart et al. (Stewart et al., Nature 2017, 549, 96-100). One tumor-bearing mouse was sacrificed when the volume of the subcutaneous tumor reached approximately 500 mm$^3$. The harvested tumor tissue (260 mg) was minced thoroughly with sterile scissors, and was subsequently enzymatically digested for 10 min at 37°C by incubating it with 1.2 $\mu$g/ml trypsin (Sigma-Aldrich) in Neurobasal-A medium (Life Technologies). The reaction was stopped by adding 1.2 $\mu$g/ml trypsin inhibitor (Sigma-Aldrich), followed by the repeated stepwise addition of 60 $\mu$l 1 mg/ml DNAse in 0.5 M MgCl$_2$ until viscosity of the solution was decreased, such that remaining tumor fragments easily settled to the bottom of the tube. After passing the cell suspension through a 40 $\mu$m cell strainer (Corning), the cells were spun down at 500g for 10 min at room temperature. Red blood cells were removed by two cycles of 2 min incubation of the cell pellet in 2 ml ACK Lysing buffer (Lonza) at room temperature, followed by washing with a TSM base medium. The cells were taken into culture in TSM complete medium and formed free-floating three-dimensional spheroids and semi-adherent spheroids within 24 hours after tumor dissociation. Free-floating and semi-adherent spheroids were dissociated with Trypl Express (Life Technologies; 5 min, 37°C) when they reached a size of 700-1000$\mu$m, and the cells were sub-cultured at a ratio of 1:3 to 1:5 in fresh TSM complete. When the cells reached cell passage six, they were considered to be established and long-term cultures (INF_R_1632_PDX_LTC); we did not observe an obvious slowdown in cell growth over at least 35 passages in culture.

*1.2 Western Blot*

Western blot analysis was performed as previously described (Oehme et al., Clin Cancer Res 2009, 15, 91-99). The primary antibodies anti-p53 (sc-126, Santa Cruz Biotechnology, Dallas, USA) and anti-GAPDH (JC1682928, Millipore, Darmstadt, Germany) were used. Peroxidase conjugated goat anti-mouse IgG (Fc specific) antibodies (A-0168, Sigma-Aldrich) were used as secondary antibodies.

*1.3 Real-Time PCR*

Real-time PCR was performed as described previously (Fischer et al., J Mol Diagn 2005, 7, 89-96; Witt et al., Blood 2003, 101, 2001-2007). The following primers were purchased from ThermoFisher Scientific: *CDKN1A* (*p21$^{WAF1/CIP1}$*, forward: 5'-TGG AGA CTC TCA GGG TCG AAA-3'(SEQ ID NO:1), reverse: 5'-GGC GTT TGG AGT GGT AGA AAT C-3'(SEQ ID NO.2)), *HPRT* (forward: 5'-TGA CAC TGG CAA AAC AAT GCA-3'(SEQ ID NO:3), reverse: 5'-GGT CCT TTT CAC CAG CAA GCT-3'(SEQ ID NO:4)), *SDHA* (forward: 5'-TGG GAA CAA GAG GGC ATC TG-3'(SEQ ID NO:5), reverse: 5'-CCA CCA CTG CAT CAA ATT CAT G-3'(SEQ ID NO:6)), and *PUMA* (forward: 5'-CCT GGA GGG TCC TGT ACA ATC T-3'(SEQ ID NO:7), reverse: 5'-GCA CCT AAT TGG GCT CCA TCT-3'(SEQ ID NO:8)). The primer for *GADD45A* was purchased from Qiagen, Hilden, Germany (QT00014084). The delta-delta-Ct method was used to calculate the relative expression, and normalization was performed to neuroblastoma specific housekeeping genes *SDHA* and *HPRT* (Fischer et al., J Mol Diagn 2005, 7, 89-96).

*1.4 Flow Cytometry Analysis*

Flow Cytometry was applied to analyze the lipid peroxidation (BODIPY staining) and to detect ROS (DCFDA staining). BODIPY 581/591 C11 Lipid peroxidation sensor staining (stock concentration: 20 mM in dimethylformamide, Invitrogen, Thermo Fisher Scientific) was used at a working concentration of 20 $\mu$M, and was diluted with RPMI without phenol red (Gibco). After inspection, the cells were detached, centrifuged and stained for 20 minutes at 37°C in a 500 $\mu$l staining solution. After centrifugation, cells were resuspended in 300 $\mu$l RPMI without phenol red, supplemented with 10% FCS. Fluorescence was measured in a FACSCanto II (BD) with 488 nm excitation and detected using a 502 nm longpass and 530/30 nm bandpass filter. Data were analyzed using FlowJo 10.x.

ROS detection was performed as previously described (Oehme et al., Proc Natl Acad Sci U S A 2013, 110, E2592-2601).

*1.5 Metabolic activity Assays*

If not otherwise declared, cells were precultured for 72 h at a density of 2,000,000 cells per T75 flask (exceptions: Kelly, NB1 and IMR-32 4,000,000 per T75 flask, SJ-GBM2 1,000,000 per T75 flask). Cells were detached with trypsin-EDTA (ThermoFisher Scientific) and seeded in 96-well plates (Greiner, Microplate, 96-Well, PS, F-bottom $\mu$CLEAR®, CELLSTAR®) in 100 $\mu$l medium per well at a density of 5,000 (KNS-42, SJ-GBM2, SF188, SK-N-BE(2)-C) or 10,000 (IMR-32, NB1, Kelly) cells/well. HDAC-inhibitor screenings were performed accordingly in 384-well plates (Greiner, Microplate, 384-Well, PS, F-bottom µCLEAR®, CELLSTAR®) in 20 µl medium per well at a density of 1,250 (SK-N-BE(2)-C, SK-N-AS) or 2,500 (IMR-32) cells/well. To avoid edge effects, margin wells (row A and H, column 1 and 12) contained PBS or medium and medium-containing wells were used as background controls. After 24 h incubation, cells were treated for 72 h. Drugs were applied using a Tecan D300e drug printer. Cell viability was quantified using CellTiter-Glo® 2.0 kit (Promega, Madison, USA). 50 µl CellTiter-Glo® (96-well) or 25 µl CellTiter-Glo® (384-well) were added to every well, the plates were shaken at 400 rpm for 5 min and were then incubated for another 10 min. Bioluminescence was quantified on an OPTIMA plate reader (BMG Labtech, Ortenberg, Germany).

*1.6 Trypan Blue Assay*

An automated Trypan Blue Assay was performed as previously described (Kolbinger et al., Arch Toxicol 2018, 92, 2649-2664).

*1.7 Zebrafish Lines*

The care for and breeding of the zebrafish were done under standardized conditions. Zebrafish wild-type AB line was raised at 28°C. Embryos used for tumor injections were maintained in an E3 buffer supplemented with 0.2 mM 1-phenyl-2- thiourea (PTU, Sigma).

*1.8 Cell preparation for zebrafish embryo xenotransplantation*

Cell preparation and xenotransplantation were performed as described previously (Wrobel et al., Pharmaceuticals (Basel) 2020, 13). Briefly, cells (NB-1 and IMR-32) were cultured to 70%-80% confluence, and then harvested and labeled with CellTracker CM-DiI (Thermo Fisher Scientific, Waltham, MA, USA). To minimize cell clumping, DNase I (250 Kunitz units/ml, Sigma-Aldrich) for IMR-32 and Benzonase (E1014-25KU, Sigma-Aldrich) for NB-1 was added to the cell suspension and washed twice with 10% FCS RPMI, twice with serum-free RPMI and resuspended in serum-free RPMI to a final concentration of $1.0 \times 10^8$ cell/ml. Zebrafish embryos were anesthetized with tricaine (MS-222, 3-Amino-benzoesäure-ethylester -methansulfonat, 0.02% (w/v), Sigma-Aldrich) and embedded in 1.0% of low gelling temperature agarose (Sigma-Aldrich). 150 to 250 CM-DiI-labeled tumor cells were injected into the yolk sac of each embryo using a FemtoJet express microinjector (Eppendorf, Hamburg, Germany) and glass microinjection needles (Science Products, Hofheim, Germany). Shortly (30 min) after injection, embryos were transferred to and held at 34°C.

*1.9 Zebrafish embryo drug toxicity assay*

The toxicity of the compounds was assessed before performing in vivo experiments and a Maximum Tolerated Dose (MTD) was determined. Embryos (48 hpf) were transferred to 48-well uncoated plates (Corning) containing different concentrations of the compounds and a solvent control. The stock solution of the compounds was further dissolved in E3 buffer supplemented with 0.2mM 1-phenyl-2- thiourea (PTU, Sigma) in order to reach the desired concentrations. Three embryos were tested per concentration or solvent control. Embryos were kept at 34°C during the experiment and were imaged at 72 hpf and 120 hpf using a stereo microscope (Leica). The embryos were assessed for signs of toxicity, like death, morphology changes (curvature of body, edema) and behavioral changes. APR-246 concentration range tested: 10-200 µM, MTD: 50µM; solvent control: DMSO (0.2% and 0.5%); Vorinostat concentration range tested: 2.5-100 µM, no toxicity detected (MTD: 100µM); solvent control: DMSO (0.1%).

*1.10 Zebrafish embryo drug treatment and efficiency evaluation*

Drug treatment and efficiency evaluation were performed as described previously (wrobel et al. 2020, loc. cit.). Briefly, embryos with tumor xenografts, detected by red signals in fluorescence microscopy (Nikon, Tokio, Japan) 2 h post implantation, were transferred to 48-well uncoated plates (Corning). Then, embryos were incubated in freshly prepared E3 medium supplemented with 1% N-Phenylthiourea (PTU, Sigma-Aldrich) containing drugs or solvent control. Tumor growth was evaluated by confocal microscopy before drug exposure and 48 h post treatment (Zeiss LSM 710 confocal microscope (Zeiss, Oberkochen, Germany) and ZEN software (Zeiss)). The responses of tumor volumes were categorized according to the Response Evaluation Criteria in Solid Tumors (RECIST) 1.1, which was adopted for zebrafish tumors, to visualize best response: progressive disease (PD, at least a 20% increase in tumor volume), stable disease (SD, neither sufficient shrinkage to qualify for PR nor sufficient increase to qualify for PD), and partial response (PR, at least a 30% decrease in tumor volume).

*1.11 In silico data analysis and genetic signature generation*

We downloaded drug response data for n=1107 cell lines to PRIMA-1 from the Cancer therapeutics response CTD[2] portal (Broad Institute, CTRP v2, 2015,). The data were combined with gene expression and *TP53* mutation data for the respective cell lines and entities from the Cancer Cell Line Encyclopedia (CCLE, Broad Institute, n=883 from 1375 CCLE cell lines had TP53 data on the CTRP); doublets were excluded. We first used drug response data to discriminate between the cell lines that were responsive (area under curve below 11, reflecting an app. IC50 of less than 10 $\mu$M, n=102 cell lines) and less-responsive (area under curve above 13, reflecting an app. IC50 of more than 40 $\mu$M, n=303 cell lines) to PRIMA-1 treatment. We then looked for differentially expressed genes in the responsive versus less-responsive cohorts using the Cancer Cell Line Encyclopedia (CCLE, Broad Institute) gene expression data set in R2 (R2: microarray and visualization platform: r2.amc.nl).

Using z-scores of log2 transformed gene expression data, we identified n=171 genes as the most significantly differentially expressed genes between the two cohorts (p-value < 2.82 $\times$ 10$^{-31}$ n=16 upregulated and n=155 downregulated genes). We tested every gene on this list for its suitability as a single gene predictive biomarker against the whole CCLE cohort. Downregulated genes were characterized by a negative overall fold change; upregulated genes were characterized by a positive overall fold change. Whether a gene in a respective cell line was up- or downregulated was reflected by its z-score. Based on how often these predictions were true, we calculated the sensitivity (true positive prediction rate), specificity (true negative prediction rate) and general false prediction rate using an R script.

To investigate the predictive power of the combinations of several genes, we first analyzed the up- and downregulated genes separately: For upregulated genes (n=16), we tested every possible combination of genes by calculating the sum of the z-scores for every cell line. We assumed that positive and negative sums of z-scores would predict the responders and low-responders, respectively. We then calculated the sensitivity, specificity and false prediction rate by comparing the number of predicted responders and low-responders to the actual responder vs. low-responder status. The best combination, with 84.3% sensitivity, 96.7% specificity and a 5.0% false prediction rate, was then used for the ratio calculation described below.

Due to the high number of downregulated genes (n=155), we analyzed a limited number of 40 downregulated genes: 20 with the lowest false prediction rate and 20 with the highest sensitivity from the single gene analysis. These 40 genes were combined in every possible way up to a group size of five. Sensitivity and specificity of every combination were calculated under the following assumptions: A negative sum of z-scores predicted responders and a positive sum of z-scores predicted low-responders. Predictions and true responder/low-responder status were compared. Based on the number of correctly predicted responder and low-responder sensitivity, specificity and false prediction rate were calculated. The best combination, with 90.2% sensitivity, 94.7% specificity and a 5.0% false prediction rate was then used for the ratio calculation.

To merge the best above identified combinations of down- and upregulated genes, we divided the sum of the upregulated gene expression values (log 2 transformed) by the sum of downregulated gene expression values (log2 transformed). To identify a cut-off value for the discrimination of responders from low-responders, we used the receiver operating curve (ROC) analysis (ROCR R-package). Samples with a ratio > ROC cut-off are predicted as responders, samples with a ratio < cut-off are predicted as low-responders. The R-package compared the prediction to real response from the Cancer Therapeutics Response Portal and calculated the sensitivity, specificity and false prediction rate.

By using the ratio of log2 transformed gene expression values of the respective genes, it was possible to calculate a genetic signature score ("biomarker") for any cohort of interest (e.g., INFORM).

*1.12 Statistical Analyses*

All cell culture experiments were performed in triplicate. A two-tailed t-test, unpaired or paired, or ANOVA where appropriate, were performed using software program R (R version 3.2.2, 2015; The R Foundation for Statistical Computing). The packages tidyverse, janitor, readxl, writxl, ROCR, ggplot2 and ggsignif were used to calculate and plot the data. P-values of less than 0.05 were considered significant (*p < 0.05, **p < 0.01, ***p < 0.001). IC50 values were calculated using GraphPad Prism Version 9.1.0 (GraphPad Software). Synergistic interactions were analyzed with SynergyFinder using the HSA (highest single agent) reference model, as the assumptions of other models were not fulfilled.

## 2. Results

*2.1 Pure TP53 mutation status is not a sufficient biomarker for APR-246 sensitivity of pediatric tumors of the nervous system*

While developed as a p53 stabilizing agent, it has emerged that *TP53's* mutation status alone is not a satisfactory biomarker for APR-246/PRIMA-1 sensitivity, e.g., in Ewing sarcoma and esophagus carcinoma models. To investigate APR-246/PRIMA-1 responsiveness and the predictive value of the *TP53* mutation status for childhood tumors, we applied in silico data analysis. We combined the data for the *TP53* mutation status of a large cohort of cancer cell lines (including pediatric entities such as medulloblastoma, neuroblastoma and Ewing sarcoma) from the Cancer Cell Line Encyclopedia (CCLE, Broad Institute) and the DepMap portal with PRIMA-1 sensitivity data obtained from the CTD[2] portal (Broad Institute, CTRP v2, 2015). If the *TP53* mutation type was the main driver of responsiveness, sensitivity to PRIMA-1/APR-246 would be expected in cell lines that harbor *TP53* point mutations leading to a conformational change impairing DNA binding, as this can be reversed by APR-246/PRIMA-1. These include the missense mutations R273H, R273C, C135F and G266E. In line with the studies mentioned above, our analysis revealed no increased sensitivity in cell lines (total as well as pediatric-only) harboring these missense mutations (Figure 1a).

Moreover, the wet-lab results from five pediatric tumor cell lines treated with APR-246 proved no correlation of IC50 values with the *TP53* mutation status. In fact, the *TP53* wild type cell line (IMR-32) was the most sensitive of all tested cell lines, as determined by metabolic activity readout (Table 1). We used three cell lines that harbor common *TP53* missense mutations within their DNA-binding domain (R273C, G266E and C135F), one cell line with a nonsense mutation (R342*, DNA-binding function remains intact), and one *TP53* wild-type cell line (Table 1). The Western blot analysis revealed the accumulation of p53 protein, which is typical for *TP53* mutation, for SJ-GBM2 (R273C), SF188 (G266E) and SK-N-BE(2)-C (C135F) cells (Figure 1b). To further investigate the contribution of p53 for APR-246 sensitivity, we treated NIH3T3-*TP53* knockout cells and the corresponding NIH3T3-*TP53* wild-type cells with APR-246. Both cell types equally responded to APR-246 treatment, independently of the *TP53* status (Table 1).

**Table 1**. Sensitivity to APR-246 in five different pediatric tumor cell models of the nervous system, measured via metabolic activity (CTG).

| Cell line | Entity / *TP53* status | IC50 / 95% CI [$\mu$M] |
|---|---|---|
| SJ-GBM2 | pHGG[1] / mut, p.R273C | 71.8 / 69.3 - 74.4 |
| SF188 | pHGG / mut, p.G266E | 43.9 / 39.4 - 48.8 |
| KNS-42 | pHGG / mut, p.R342*[2] | 27.8 / 22.8 - 33.7 |
| SK-N-BE(2)-C | neuroblastoma / mut, p.C135F | 24.7 / 22.0 -27.7 |
| IMR-32 | neuroblastoma / wt | 4.0 / 3.2 - 4.8 |
| NIH-3T3 C5-GL (*TP53* ko) | murine embryo fibroblasts | 18.4 / 15.8 - 21.3 |
| NIH-3T3 □6RT (*TP53* wt) | murine embryo fibroblasts | 18.7 / 17.6 - 19.9 |

[1] pediatric high-grade glioma
[2] nonsense mutation affecting the tetramerization domain, not the DNA binding domain of p53

To determine whether APR-246 exerts its p53-activating potential, we combined APR-246 with doxorubicin, which is a well-known DNA damage and p53 response inducer. Doxorubicin single-treatment activated p53 in the *TP53*-wt cell line IMR-32, as indicated by the upregulation of the p53 target genes *CDKN1A, PUMA* and *GADD45A* (Figure 1c). The addition of APR-246 to doxorubicin treatment did not increase target gene expression in the *TP53*-wildtype setting when compared to cells treated with doxorubicin only (Figure 1c). In the *TP53* mutant neuroblastoma cell line SK-N-BE(2)-C (C135F), doxorubicin single treatment failed to activate p53 target gene expression. Furthermore, the addition of APR-246 to doxorubicin did not improve p53 target gene induction in this cell line (Figure Id). Nevertheless, when combining a fixed APR-246 concentration with different doses of doxorubicin, we observed increased doxorubicin sensitivity in both the *TP53*-wt IMR-32 cell line and in the doxorubicin resistant *TP53*-mutant SK-B-BE(2)-C cell line. Our metabolic activity data thus suggest that the combination of APR-246 with doxorubicin reduces viability in both *TP53*-wt and mutant neuroblastoma cell lines, despite the absence of a p53 target gene expression response in the *TP53*-mutant cell line.

These findings were confirmed in *TP53* mutant pHGG cell lines SJ-GBM2 and SF188. Similar to SK-N-BE(2)-C cells, both cell lines exhibited decreased cell viability upon doxorubicin treatment combined with APR-246. Again, we observed no reactivation of p53 target gene expression (*CDKN1A* and *GADD45A*) upon APR-246 treatment in these cells. Altogether, these results suggest that the *TP53* mutation status alone is not sufficient to predict the response to APR-246 alone or in combination with doxorubicin in childhood tumors of the nervous system.

*2.2. APR-246 impairs oxygen species elimination and basal reactive oxygen species level indicate APR-246 sensitivity*

Besides binding to mutant p53, the APR-246/PRIMA-1 product MQ has been described to covalently bind to and inactivate the antioxidant tripeptide glutathione. Additionally, MQ has been shown to inhibit thioredoxin reductase 1 (TrxR1) resulting in a pro-oxidant NADPH-oxidase. These effects result in increased levels of reactive oxygen species (ROS). We observed increased ROS formation and lipid peroxidation in our neuroblastoma models upon APR-246 treatment (Figure 2a-b). In addition, we compared basal ROS levels of responsive IMR-32 as well as less responsive SK-N-BE(2)-C and SJ-GBM2 cells. Whereas the sensitive IMR-32 cells displayed very high basal ROS levels, the less-responsive cell lines exhibited only low basal ROS levels, suggesting that cells with a high degree of basal oxidative stress respond better to the treatment (Figure 2c). To test this assumption, we analyzed a vincristine resistant and vincristine non-resistant neuroblastoma cell line pair, generated through long-term cultivation of the parental cell line in vincristine or solvent containing medium. Consistent with our hypothesis, the chemotherapy resistant cell model displayed significantly increased ROS-levels, and in turn increased APR-246 sensitivity, when compared to its chemotherapy non-resistant control counterpart (Figure 2d-e).

ROS levels are controlled by intracellular glutathione levels and cysteine importers, such as the cystine-glutamate antiporter encoded by the *SLC7A11* gene. Consequently, low *SLC7A11* expression levels correlate with low amounts of glutathione and high ROS levels. Low amounts of glutathione are completely bound by APR-246/MQ and the expression of *SLC7A11* has been proposed as a predictive biomarker of APR-246 responsiveness in esophagus carcinoma cell lines (Liu et al. 2017, loc. cit.).

To test *SLC7A11* as a response prediction marker for APR-246 sensitivity in pediatric nervous system tumors, we first performed an in silico data analysis of responsive and less-responsive cell lines of the whole Cancer Cell Line Encyclopedia (CCLE), including all cell lines. We divided the cell lines into two groups according to their sensitivity. Based on pharmacokinetics data, we defined the maximum APR-246 trough plasma level of 20 $\mu$M in order to discriminate between responders and low-responders. We then analyzed the *SLC7A11* gene expression in the two CCLE groups. Indeed, *SLC7A11* expression was significantly lower in responsive versus low-responsive cell lines (Figure 2f).

In a reverse approach, we investigated which cell lines from which entities displayed low *SLC7A11* expressions. In particular, cell lines of the pediatric entities neuroblastoma and acute lymphoblastic leukemia, as well as adult entities lymphoma and acute myeloid leukemia, expressed *SLC7A11* at a low level. We hypothesized that the low *SLC7A11* expression was associated with a higher responsiveness of these entities to APR-246/PRIMA-1. Combining the expression and sensitivity data confirmed this assumption (Figure 2g, Figure S5). Moreover, *SLC7A11* expression levels of each entity correlated well with their mean APR-246 sensitivity

(Pearson correlation coefficient of 0.725) (Figure 2h). Taken together, a low *SLC7A11* expression indicates a high APR-246/PRIMA-1 responsiveness.

Analyzing expression data from the Individualized Therapy For Relapsed Malignancies in Childhood (INFORM) register study using the R2 bioinformatic data analysis platform, we could confirm that neuroblastoma patient samples sensitive to APR-246/PRIMA-1 are characterized by very low *SLC7A11* levels, as compared to most other pediatric entities. Notably, acute lymphoblastic lymphoma (ALL) and non-Hodgkin lymphoma (NHL) showed a similarly low expression (Figure 2i).

To verify our in silico analysis with wet-lab data, we tested seven randomly picked cell line models (four neuroblastoma, two ALL and one non-Hodgkin lymphoma (NHL) cell lines) for APR-246 sensitivity. Six of the seven models were highly responsive to treatment with APR-246 (Table 2).

**Table 2.** Sensitivity to APR-246 in seven different models.

| Cell line | Entity | IC50 / 95% CI [$\mu$M] | *SLC7A11* expression[3] |
|---|---|---|---|
| Kelly | Neuroblastoma | 8.48 / 6.8 - 10.0 | 1.43 |
| NB-1 | Neuroblastoma | 4.83 / 4.22 - 5.42 | 1.35 |
| SH-EP | Neuroblastoma | 44.5 / 40.1 - 49.5 | - |
| SK-N-FI | Neuroblastoma | 9.21 / 8.02 - 10.1 | 0.88 |
| NALM-6 | ALL[1] | 4.56/3.09 - 6.85 | 2.51 |
| REH | ALL[1] | 4.39 / 4.0 - 4.84 | 1.41 |
| HuT 78 | NHL[2] | 9.47 / 8.23 - 10.74 | 1.45 |

[1] acute lymphoblastic leukemia

[2] non-Hodgkin lymphoma

[3] Log2 values, derived from CCLE, SH-EP are not included in CCLE

Taken together our data show that APR-246 treatment results in oxidative stress and cell death and that oxidative stress sensitizes cells to APR-246 treatment. A low *SLC7A11* expression indicates a high APR-246 responsiveness. Entities with rather low *SLC7A11* expression levels (CCLE data) are neuroblastoma, ALL or NHL. A similar *SLC7A11* expression pattern was observed in the INFORM patient cohort, with ALL, rhabdomyosarcoma, NHL and neuroblastoma being those with the lowest SLC7A11 expressions.

*2.3 A novel biomarker signature improves response prediction for neuroblastoma cell lines and patients*

While *SLC7A11* indicates responsiveness quite well for tumor entities, we noticed that the correlation between *SLC7A11* expression and APR-246/PRIMA-1 sensitivity (IC50) was reduced in neuroblastoma cell lines (Pearson correlation coefficient of 0.145, Figure 3a).

To screen for additional novel APR-246/PRIMA-1 response prediction biomarkers in neuroblastoma we defined two response groups based on the 20 $\mu$M plasma trough level threshold: high responders (app. IC50 < 10 $\mu$M, 50% plasma trough level) and low-responders (app. IC50 > 40 $\mu$M, 200% plasma trough level). We screened R2 gene expression data sets (CCLE, including e.g. data from acute myeloid leukaemia, acute lymphoblastic leukaemia, acute lymphoblastic B-cell leukaemia, breast cancer (ductal carcinoma), lung cancer (small cell lung carcinoma), liver cancer (hepatocellular carcinoma), large intestine cancer (adenocarcinoma), and lymphoma (diffuse large B-cell lymphoma and hodgkin lymphoma)) for genes that are differently expressed between high and low-responders (Figure 3b). Among the 171 most significantly differentially expressed genes 155 genes showed a negative fold change from low-responders to high responders, meaning that these genes were downregulated in responders; 16 genes showing a positive fold change were accordingly upregulated in responders.

The top downregulated gene indicating APR-246 sensitivity, was *YAP1* (p-value $1.08 \times 10^{-69}$, fold change -2.32, Figure 3c). The top upregulated gene indicating sensitivity was *MIR142* (p-value $1.28 \times 10^{-58}$, fold change 17.75, Figure 3d). We tested the predictive value of the identified genes to classify high-responder/low-responder, resulting in: 79.4% (*YAP1*) and 73.5 % *(MIR142)* sensitivity and 84.8% (*YAP1*) and 86.1% (*MIR142*) specificity. To generate a biomarker with improved sensitivity and specificity, we combined highly differentially expressed genes to the following gene signature and calculated a signature score according to this formula (sum of upregulated genes divided by sum of downregulated genes):

$$(\log 2 CORO1A + \log 2 RHOH + \log 2 CXCR4 + \log 2 PUM2 + \log 2 TRAF3IP3 + \log 2 KDM2B + \log 2 PDE7A + \log 2 ARHGAP9) \, /$$

$$(\log 2 PLS3 + \log 2 RHBDF1 + \log 2 CLDN1 + \log 2 SLC7A11 + \log 2 FAM114A1)$$

The optimal cut-off value for a biomarker discriminating between a high responder and low-responder was 1.1, as determined with a receiver operating characteristic (ROC) curve. Higher values indicate higher responsive samples and lower values indicate less responsive samples. The sensitivity at this cut-off was 90.0% and the specificity was 94.3% (Figure 3e). In the next step, we applied the identified gene signature to all cell lines in the CCLE with available sensitivity data and chose the maximum APR-246 plasma trough level of 20 $\mu$M as the sensitivity threshold to be predicted. With this threshold, the most efficient biomarker cut-off was determined to be 1.0, giving a sensitivity of 71.5% and a specificity of 83.8% (Figure 3f). With this biomarker at hand, we again investigated the neuroblastoma sub-cohort of the CCLE and found that 10 out of the 12 models were correctly predicted (Figure 3g).

We further challenged the biomarker's predictions and acquired our own cell culture data from three neuroblastoma models with known expression data, but with unknown sensitivity: SIMA (biomarker 1.80), SH-SY5Y (biomarker 1.68) and SK-N-AS (biomarker 1.03). We expected a cell line with a signature score higher than 1.0 to have an IC50 of below 20 $\mu$M and vice versa. The criteria were met in two of three neuroblastoma cell lines. We also tested three more models of other entities as well as four cultures derived from INFORM patient samples. Notably, the neuroblastoma long-term culture was derived from a neuroblastoma PDX model of an INFORM patient. We identified the APR-246 sensitivity and compared it to the calculated genetic signature score. All three established cell lines (KNS-42, Jurkat, JeKo-1) met the criteria, and three of the four INFORM samples were correctly predicted (Table 3).

**Table 3**. Biomarker validation in ten randomly picked samples (>1.0 predicts sensitivity; sensitivity is defined as IC50 < 20 $\mu$M).

| Model Type | Name / Entity | Biomark er | IC50 / 95% CI [$\mu$M] |
|---|---|---|---|
| Established cell lines | SK-N-AS / neuroblastoma | 1.03 | 35.1 / 29.2 - 43.1 |
| | SIMA / neuroblastoma | 1.80 | 2.04 / 1.74 - 2.4 |
| | SH-SY5Y / neuroblastoma | 1.68 | 19.7 / 16.8 - 22.4 |
| | KNS-42 / pHGG | 0.90 | 27.8 / 22.8 - 33.7 |
| | Jurkat / ALL | 3.20 | 1.46 / 1.38 - 1.54 |
| | JeKo-1 / NHL | 5.78 | 1.44 / 1.32 - 1.58 |

(continued)

| Model Type | Name / Entity | Biomark er | IC50 / 95% CI [$\mu$M] |
|---|---|---|---|
| INFORM long-term cultures | INF R 1288 LTC / rhabdoid tumor | 1.09 | 19.7/ 14.9 - 25.8 |
| | INF_R_1467_LTC / soft tissue sarcoma | 1.58 | 18.4 / 16.9 - 20.0 |
| | INF_R_1490 LTC / osteosarcoma | 1.19 | 28.7 / 25.5 - 32.0 |
| INFORM-PDX long-term culture | INF_R_1632_PDX_LTC/ neuroblastoma | 3.66 | 12.2 / 8.7 - 16.6 |

Notably, only 35.5% of all cell lines in the CCLE (n=1375) were predicted to be responders according to our signature score. In contrast, 90.0% of CCLE neuroblastoma cell lines were predicted to be responsive, again highlighting the pronounced sensitivity of this entity to APR-246 treatment.

Further biomarker combinations

To establish that the method specified herein can also be put into practice with a lower number of biomarkers, combinations of four or more biomarkers were tested, e.g. the following:
A score of (log2IKZF1+ log2KDM2B) / (log2PLS3 + log2TJP1) provided a sensitivity of 71.1% and a specificity of 77.0% for all tumor types. A score of (log2PUM2+ log2TRAF3IP3+ log2ACAP1 + log2PDE7A) / (log2SEPTIN10+ log2YAP1+ log2TJP1+ log2SLC7A11) provided a sensitivity of 71.1% and a specificity of 79.1% for all tumor types.
For the case of AML, a four biomarker score of (log2IKZF1+ log2KDM2B) / (log2PLS3 + log2TJP1) provided a sensitivity of 61% and a specificity of 72%. Also for the case of AML, an eight biomarker score of (log2PUM2+ log2TRAF3IP3+ log2ACAP1 + log2PDE7A) / (log2SEPTIN10+ log2YAP1+ log2TJP1+ log2SLC7A11) provided a sensitivity of 78% and a specificity of 82%.

*3.4. Sensitzing low-responsive neuroblastoma cells to APR-246 using HDAC-Inhibitors*

Despite the generally high responsiveness, our analysis revealed that about 10% of neuroblastoma models were predicted to be less responsive to APR-246. As increased ROS levels augment the effect of APR-246 treatment, we assessed whether we could sensitize low-responsive cells to APR-246 treatment by ROS induction, e.g., by treatment with HDAC inhibitors. HDAC inhibitors are known ROS inducers. We pretreated with 0.5 $\mu$M vorinostat for 72 h before addition of 20 $\mu$M APR-246 for 24 h and measured cell viability and cell death via trypan blue exclusion assay (Figure 4a-d). Consistent with our hypothesis, both compounds alone showed only minor effects, whereas the combination resulted in a significant reduction in the viable cell number and a significant increase in dead cells in APR-246 low-responsive SK-N-AS and SK-N-BE(2)-C cells.
Similar results were obtained with the HDACis abexinostat, romidepsin and panobinostat in SK-N-AS, SK-N-BE(2)-C and IMR-32 cells (Table 4 below). For less responsive SK-N-AS cells at 72 h, co-treatment with HDACi vorinostat resulted in a HSA synergy score of 12.17, which indicated a synergistic effect of the drug combination (Figure 4e, f)). In line with our hypothesis, HDACi treatment increased ROS levels in IMR-32 cells and less responsive SK-N-AS cells had a lower basal ROS level compared to responsive IMR-32 cells.
To test the combination of the HDAC inhibitor and APR-246 in vivo, we used zebrafish embryo neuroblastoma xenograft models of NB-1 and IMR-32 cells. Fluorescently labeled tumor cells were transplanted into the yolk sack on day two post fertilization. Treatment with 50 $\mu$M APR-246, 1.5 $\mu$M vorinostat, solvent control and the combination was started on day three post fertilization. In both models decreased tumor growth was observed under treatment with single compounds. However, in both cases the combination was more potent, underlining the effectiveness of combining HDAC inhibition and APR-246 treatment in neuroblastoma models (Figure 4g-n). Taken together, these results demonstrate that combining HDAC inhibition with ROS induction via APR-246 is effective in the treatment of neuroblastoma in vitro and in vivo.
Literature:

Fischer et al., J Mol Diagn 2005, 7, 89-96
Koeneke et al., Cells 2015, 4, 135-168
Kolbinger et al., Arch Toxicol 2018, 92, 2649-2664

Liu et al., Nat Commun 2017, 8, 14844

Oehme et al., Clin Cancer Res 2009, 15, 91-99

Oehme et al., Proc Natl Acad Sci U S A 2013, 110, E2592-2601

Peng et al. Cell death & disease 2013, 4, e881-e881

Ridinger et al., Scientific reports 2018, 8, 10039

Stewart et al., Nature 2017, 549, 96-100

Witt et al., Blood 2003, 101, 2001-2007

Wrobel et al., Pharmaceuticals (Basel) 2020, 13

**Table 4**. HDACi screening for abexinostat, romidepsin and panobinostat in three neuroblastoma cell line models.

| Cell line | HDAC inhibitor | IC50 [nM] | 95% CI [nM] | + APR-246 [µM] | IC50 [nM] | 95% CI [nM] |
|---|---|---|---|---|---|---|
| SK-N-AS | abexinostat | 46.3 | 29.0 - 69.8 | 20 | 15.4 | 9.7 - 23.8 |
| | romidepsin | 5.7 | 4.9 - 6.6 | 20 | 1.8 | 1.5 - 2.2 |
| | panobinostat | 2.2 | 1.6-2.9 | 20 | 1.0 | 0.6 - 1.4 |
| SK-N-BE (2)-C | abexinostat | 172.1 | 142.2 - 207.5 | 10 | 37.5 | 22.5 - 58.9 |
| | romidepsin | 26.9 | 15.2 - 140.9 | 10 | 9.9 | 3.6 - 52.9 |
| | panobinostat | 40.2 | 29.4 - 54.6 | 20 | 3.1 | 0.3 - 12.2 |
| IMR-32 | abexinostat | 100.0 | 56.3 - 169.7 | 2 | 30.4 | 6.6 - 48.9 |
| | romidepsin | 0.73 | 0.64 - 0.83 | 1 | 0.24 | 0.13 - 0.33 |
| | panobinostat | 35.6 | 29.0 - 43.7 | 2 | 13.9 | 11.0 - 17.2 |

**Claims**

1. A method for identifying a subject susceptible to treatment of cancer by administration of a methylene quinuclidinone agent, said method comprising

   (a) determining in a sample of said subject at least four biomarkers independently selected from the group consisting of SLC7A11, PLS3, RHBDF1, CLDN1, FAM114A1, YAP1, TJP1, SEPTIN10; COROIA, RHOH, CXCR4, PUM2, TRAF3IP3, KDM2B, PDE7A, ARHGAP9, IKZF1, ACAP1, and PDE7A;
   (b) comparing the result determined in step (a) to at least one reference, and
   (c), based on the result of step (b), identifying a subject susceptible to treatment of cancer by administration of a methylene quinuclidinone agent.

2. The method of claim 1, wherein said at least four biomarkers are selected by independently selecting at least two biomarkers from the group consisting of SLC7A11, PLS3, RHBDF1, CLDN1, FAM114A1, YAP1, TJP1, and SEPTIN10, (group 1 biomarkers); and independently selecting at least two biomarkers from the group consisting of COROIA, RHOH, CXCR4, PUM2, TRAF3IP3, KDM2B, PDE7A, ARHGAP9, IKZF1, ACAP1, and PDE7A (group 2 biomarkers).

3. The method of claim 1 or 2, wherein said method comprises

   (i) determining the biomarkers COROIA, RHOH, CXCR4, PUM2, TRAF3IP3, KDM2B, PDE7A, ARHGAP9, PLS3, RHBDF1, CLDN1, SLC7A11, and FAM114A1;
   (ii) determining the biomarkers IKZF1, KDM2B, PLS3, and TJP1; or
   (iii) determining the biomarkers PUM2, TRAF3IP3, ACAP1, PDE7A, SEPTIN10, YAP1, TJP1, and SLC7A11.

4. The method of any one of claims 1 to 3, wherein said methylene quinuclidinone agent is APR-246 (Eprenetapopt, 2-(Hydroxymethyl)-2-(methoxymethyl)-1-azabicyclo[2.2.2]octan-3-one, CAS No. 5291-32-7) or Prima-1 (2,2-bis(hydroxymethyl)-1-azabicyclo[2.2.2]octan-3-one, CAS No. 5608-24-2), preferably is APR-246.

5. The method of any one of claims 1 to 4, wherein said cancer is neuroblastoma, lymphoma, acute lymphocytic leukemia, acute myeloid leukemia, or myeloid dysplastic syndrome.

6.  The method of any one of claims 1 to 5, wherein said reference is derived from a population of cells not susceptible to treatment of cancer by administration of a methylene quinuclidinone agent,
    preferably wherein a decrease of group 1 biomarkers and/or increase of group 2 biomarkers compared to said reference is indicative of a subject susceptible to treatment of cancer by administration of a methylene quinuclidinone agent, and, optionally, wherein values of the biomarkers essentially identical to the reference are indicative of a subject not susceptible to treatment of cancer by administration of a methylene quinuclidinone agent.

7.  The method of any one of claims 1 to 6, wherein said reference is derived from a population of cells susceptible to treatment of cancer by administration of a methylene quinuclidinone agent,
    preferably wherein values of the biomarkers essentially identical to the reference are indicative of a subject susceptible to treatment of cancer by administration of a methylene quinuclidinone agent, and, optionally, wherein an increase of group 1 biomarkers and/or a decrease of group 2 biomarkers compared to said reference is indicative of a subject not susceptible to treatment of cancer by administration of a methylene quinuclidinone agent.

8.  The method of any one of claims 1 to 7, wherein said method comprises calculating a score based on the results of the determination in step (a),
    preferably wherein calculating said score comprises summing up the log2-transformed values determined for group 1 biomarkers and/or the log2-transformed values determined for group 2 biomarkers.

9.  The method of claim 8, wherein the biomarkers COROIA, RHOH, CXCR4, PUM2, TRAF3IP3, KDM2B, PDE7A, ARHGAP9, PLS3, RHBDF1, CLDN1, SLC7A11, and FAM114A1 are determined and wherein the score is calculated according to equation (1):

$$\text{Score} = (\log2\text{CORO1A} + \log2\text{RHOH} + \log2\text{CXCR4} + \log2\text{PUM2} + \log2\text{TRAF3IP3} + \log2\text{KDM2B} + \log2\text{PDE7A} + \log2\text{ARHGAP9}) / (\log2\text{PLS3} + \log2\text{RHBDF1} + \log2\text{CLDN1} + \log2\text{SLC7A11} + \log2\text{FAM114A1}) \quad (1).$$

10. The method of any one of claims 1 to 9, wherein the subject identified to be not susceptible to treatment of cancer by administration of a methylene quinuclidinone agent is identified as a subject susceptible to combined treatment with (i) a methylene quinuclidinone agent and (ii) a histone deacetylase (HDAC) inhibitor.

11. The method of any one of claims 1 to 10, wherein said sample is a cancer sample, preferably a tumor sample.

12. A methylene quinuclidinone agent for use in treatment of cancer in a subject, wherein said subject was identified to be susceptible to cancer treatment with a methylene quinuclidinone agent by the method according to any one of claims 1 to 11.

13. A combined preparation comprising (i) a methylene quinuclidinone agent and (ii) an HDAC inhibitor for use in treatment of cancer in a subject, wherein said subject was identified to not be susceptible to cancer treatment with a methylene quinuclidinone agent by the method according to any one of claims 1 to 11.

14. A kit comprising means for determining at least four biomarkers independently selected from the group consisting of CORO1A, RHOH, CXCR4, PUM2, TRAF3IP3, KDM2B, PDE7A, ARHGAP9, IKZF1, ACAP1, PDE7A; PLS3, RHBDF1, CLDN1, SLC7A11, FAM114A1, TJP1, SEPTIN10, and YAP1, preferably comprised in a housing.

15. A device comprising an evaluation unit with a processor, and, preferably tangibly embedded, instructions which, when performed on the processor, cause the device to perform at least step (b) of the method according to any one of claims 1 to 11.

Fig. 1

EP 4 134 671 A1

Fig. 2

Fig. 2 (continued)

Fig. 3

EP 4 134 671 A1

Fig. 3 (continued)

Fig. 3 (continued)

EP 4 134 671 A1

Fig. 4

Fig. 4 (continued)

EP 4 134 671 A1

Europäisches
Patentamt

European
Patent Office

Office européen
des brevets

# EUROPEAN SEARCH REPORT

**Application Number**

EP 21 19 1371

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DAVID S. LIU ET AL: NATURE COMMUNICATIONS, vol. 8, 28 March 2017 (2017-03-28), page 14844, XP055518876, DOI: 10.1038/ncomms14844 * whole document, in particular abstract; p. 2, col. 2 - p. 4, col. 1, bridging par.; fig. 3 * | 1-8, 10-15 | INV. G01N33/574 A61K31/00 |
| A | VAN GRONINGEN TIM ET AL: "A NOTCH feed-forward loop drives reprogramming from adrenergic to mesenchymal state in neuroblastoma", NATURE COMMUNICATIONS, vol. 10, no. 1, 1 December 2019 (2019-12-01), XP055885861, DOI: 10.1038/s41467-019-09470-w Retrieved from the Internet: URL:https://www.nature.com/articles/s41467-019-09470-w.pdf> * the whole document * | 1-15 | |
| A | DINA ALI ET AL: "Anti-leukaemic effects induced by APR-246 are dependent on induction of oxidative stress and the NFE2L2/HMOX1 axis that can be targeted by PI3K and mTOR inhibitors in acute myeloid leukaemia cells", BRITISH JOURNAL OF HAEMATOLOGY, JOHN WILEY, HOBOKEN, USA, vol. 174, no. 1, 15 March 2016 (2016-03-15), pages 117-126, XP071107169, ISSN: 0007-1048, DOI: 10.1111/BJH.14036 * the whole document * | 1-15 | TECHNICAL FIELDS SEARCHED (IPC) G01N A61K |
| A | WO 2017/159877 A1 (EISAI R&D MAN CO LTD [JP]) 21 September 2017 (2017-09-21) * the whole document * | 1-15 | |

The present search report has been drawn up for all claims

| Place of search | Date of completion of the search | Examiner |
|---|---|---|
| The Hague | 2 February 2022 | Chrétien, Eva Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 19 1371

This annex lists the patent family members relating to the patent documents cited in the above-mentioned European search report.
The members are as contained in the European Patent Office EDP file on
The European Patent Office is in no way liable for these particulars which are merely given for the purpose of information.

02-02-2022

| Patent document cited in search report | Publication date | Patent family member(s) | Publication date |
|---|---|---|---|
| WO 2017159877 A1 | 21-09-2017 | NONE | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PENG et al.** *Cell death & disease,* 2013, vol. 4, e881-e881
- **LIU et al.** *Nat Commun,* 2017, vol. 8, 14844
- **KOENEKE et al.** *Cells,* 2015, vol. 4, 135-168
- **RIDINGER et al.** *Scientific reports,* 2018, vol. 8, 10039
- **OEHME et al.** *Proc Natl Acad Sci U S A,* 2013, vol. 110, E2592-2601
- *CHEMICAL ABSTRACTS,* 207556-03-4
- *CHEMICAL ABSTRACTS,* 5291-32-7
- *CHEMICAL ABSTRACTS,* 5608-24-2
- **ZWEIG.** *Clin. Chem.,* 1993, vol. 39, 561
- *CHEMICAL ABSTRACTS,* 149647-78-9
- *CHEMICAL ABSTRACTS,* 404950-80-7
- *CHEMICAL ABSTRACTS,* 209783-80-2
- *CHEMICAL ABSTRACTS,* 783355-60-2
- *CHEMICAL ABSTRACTS,* 128517-07-7
- **STEWART et al.** *Nature,* 2017, vol. 549, 96-100
- **OEHME et al.** *Clin Cancer Res,* 2009, vol. 15, 91-99
- **FISCHER et al.** *J Mol Diagn,* 2005, vol. 7, 89-96
- **WITT et al.** *Blood,* 2003, vol. 101, 2001-2007
- **KOLBINGER et al.** *Arch Toxicol,* 2018, vol. 92, 2649-2664
- **WROBEL et al.** *Pharmaceuticals (Basel),* 2020, vol. 13